# EUROPEAN PATENT SPECIFICATION

(11) **EP 1 136 556 B1**
(45) Date of publication and mention of the grant of the patent: **08.06.2005**
(21) Application number: 01109203.8
(22) Date of filing: 20.11.1992
(51) Int. Cl.: C12N 15/13, C07K 16/46, C12N 15/62

(54) **Method of producing multivalent antigen-binding proteins**
Verfahren zur Herstellung von multivalenten antigenbindenden Proteinen
Procédé pour produire de protéines multivalents de fixation de l'antigène

(30) Priority: 25.11.1991 US 796936
(43) Date of publication of application: 26.09.2001
(62) Divisional of application: 93900545.0
(73) Proprietor: ENZON, INC., Piscataway, NJ 08854 (US)
(72) Inventor: Whitlow, Marc D., Gaithersburg, MD20879 (US); Wood, James F., Germantown, MD 20874 (US); Hardman, Karl, Wynnewood, PA 19096 (US); Bird, Robert E., Rockville, MD 20853 (US); Filpula, David, Gaithersburg, MD 20877 (US); Rollence, Michele, Court, Damascus, MD 20872 (US)
(74) Representative: Froud, Clive

(56) References cited:
- WO-A-91/19739
- PANTOLIANO ET AL.: "Conformational stability, folding, and ligand-binding affinity of single-chain Fv immunoglobulin fragments expressed in Escherichia coli" BIOCHEMISTRY, vol. 30, no. 42, 22 October 1991 (1991-10-22), pages 10117-10125, XP002169042 EASTON, PA US
- COLCHER D ET AL.: "In vivo tumor targeting of a recombinant single-chain antigen-binding protein" JOURNAL OF THE NATIONAL CANCER INSTITUTE, vol. 82, no. 14, 18 July 1990 (1990-07-18), pages 1191-1197, XP000574245
- MURARO R ET AL: "GENERATION AND CHARACTERIZATION OF B72.3 SECOND GENERATION MONOCLONAL ANTIBODIES REACTIVE WITH THE TUMOR-ASSOCIATED GLYCOPROTEIN 72 ANTIGEN" CANCER RESEARCH, vol. 48, no. 16, 1988, pages 4588-4596, XP001002699 ISSN: 0008-5472
- BIRD R E ET AL: "SINGLE-CHAIN ANTIGEN-BINDING PROTEINS" SCIENCE,US,AMERICAN ASSOCIATION FOR THE ADVANCEMENT OF SCIENCE, vol. 242, 21 October 1988 (1988-10-21), pages 423-426, XP000575094 ISSN: 0036-8075
- WHITLOW M ET AL.: "Multivalent Fvs: characterization of single-chain Fv oligomers and preparation of a bispecific Fv" PROTEIN ENGINEERING, vol. 7, no. 8, 1 August 1994 (1994-08-01), pages 1017-1026, XP000445076 ENGLAND GB
- PATENT ABSTRACTS OF JAPAN A

## Description

### Background of the Invention

### 1. Field of the Invention

This invention relates to a method of producing multivalent antigen-binding proteins.

### 2. Description of the Background Art

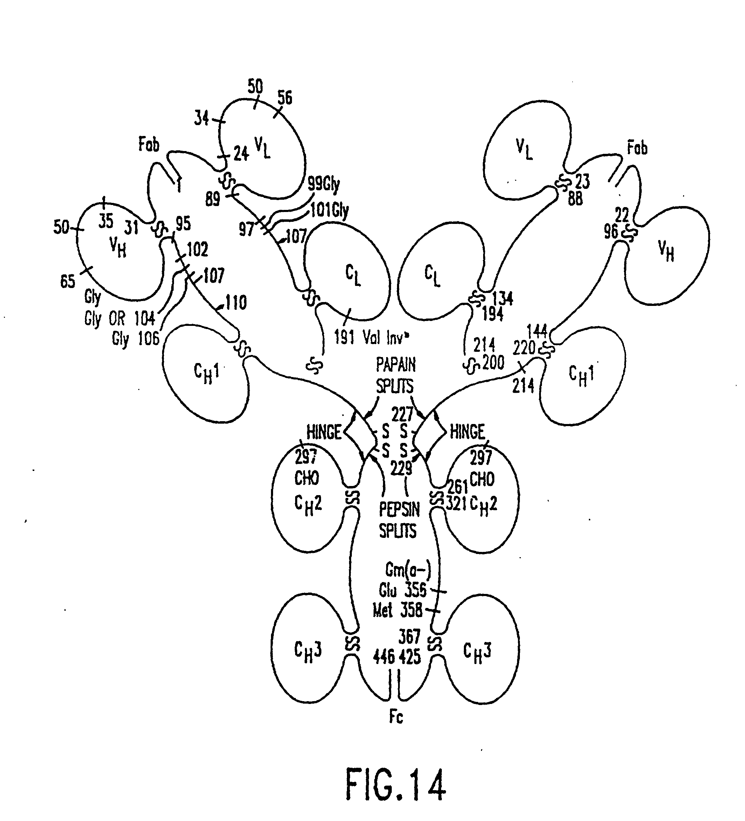

Antibodies are proteins generated by the immune system to provide a specific molecule capable of complexing with an invading molecule, termed an antigen. Figure 14 shows the structure of a typical antibody molecule. Natural antibodies have two identical antigen-binding sites, both of which are specific to a particular antigen. The antibody molecule "recognizes" the antigen by complexing its antigen-binding sites with areas of the antigen termed epitopes. The epitopes fit into the conformational architecture of the antigen-binding sites of the antibody, enabling the antibody to bind to the antigen.

The antibody molecule is composed of two identical heavy and two identical light polypeptide chains, held together by interchain disulfide bonds (see Fig. 14). The remainder of this discussion will refer only to one light/heavy pair of chains, as each light/heavy pair is identical. Each individual light and heavy chain folds into regions of approximately 110 amino acids, assuming a conserved three-dimensional conformation. The light chain comprises one variable region (termed V_{L}) and one constant region (C_{L}), while the heavy chain comprises one variable region (V_{H}) and three constant regions (C_{H}1, C_{H}2 and C_{H}3). Pairs of regions associate to form discrete structures as shown in Figure 14. In particular, the light and heavy chain variable regions, V_{L} and V_{H}, associate to form an "F_{V}" area which contains the antigen-binding site.

The variable regions of both heavy and light chains show considerable variability in structure and amino acid composition from one antibody molecule to another, whereas the constant regions show little variability. The term "variable" as used in this specification refers to the diverse nature of the amino acid sequences of the antibody heavy and light chain variable regions. Each antibody recognizes and binds antigen through the binding site defined by the association of the heavy and light chain variable regions into an F_{V} area. The light-chain variable region V_{L} and the heavy-chain variable region V_{H} of a particular antibody molecule have specific amino acid sequences that allow the antigen-binding site to assume a conformation that binds to the antigen epitope recognized by that particular antibody.

Within the variable regions are found regions in which the amino acid sequence is extremely variable from one antibody to another. Three of these so-called "hypervariable" regions or "complementarity-determining regions" (CDR's) are found in each of the light and heavy chains. The three CDR's from a light chain and the three CDR's from a corresponding heavy chain form the antigen-binding site.

Cleavage of the naturally-occurring antibody molecule with the proteolytic enzyme papain generates fragments which retain their antigen-binding site. These fragments, commonly known as Fab's (for Fragment, antigen binding site) are composed of the C_{L}, V_{L}, C_{H}1 and V_{H} regions of the antibody. In the Fab the light chain and the fragment of the heavy chain are covalently linked by a disulfide linkage.

Recent advantages in immunobiology, recombinant DNA technology, and computer science have allowed the creation of single polypeptide chain molecules that bind antigen. These single-chain antigen-binding molecules incorporate a linker polypeptide to bridge the individual variable regions, V_{L} and V_{H}, into a single polypeptide chain. A computer-assisted method for linker design is described more particularly in U.S. Patent No. 4,704,692, issued to Ladner *et al*. in November, 1987. A description of the theory and production of single-chain antigen-binding proteins is found in U.S. Patent No. 4,946,778 (Ladner *et al*.), issued August 7, 1990. The single-chain antigen-binding proteins produced under the process recited in U.S. Patent 4,946,778 have binding specificity and affinity substantially similar to that of the corresponding Fab fragment.

Bifunctional, or bispecific, antibodies have antigen binding sites of different specificities. Bispecific antibodies have been generated to deliver cells, cytotoxins, or drugs to specific sites. An important use has been to deliver host cytotoxic cells, such as natural killer or cytotoxic T cells, to specific cellular targets. (U.D. Staerz, O. Kanagawa, M.J. Bevan, *Nature* 314:628 (1985); S. Songilvilai, P.J. Lachmann, *Clin. Exp. Immunol.* 79:315 (1990).) Another important use has been to deliver cytotoxic proteins to specific cellular targets. (V. Raso, T. Griffin, *Cancer Res.* 41:2073 (1981); S. Honda, Y. Ichimori, S. Iwasa, *Cytotechnology* 4:59 (1990).) Another important use has been to deliver anti-cancer non-protein drugs to specific cellular targets (J. Corvalan, W. Smith, V. Gore, *Intl. J. Cancer Suppl.* 2:22 (1988); M. Pimm *et al*., *British J*. *of Cancer* 61:508 (1990)). Such bispecific antibodies have been prepared by chemical cross-linking (M. Brennan *et al., Science* 229:81 (1985)), disulfide exchange, or the production of hybrid-hybridomas (quadromas). Quadromas are constructed by fusing hybridomas that secrete two different types of antibodies against two different antigens (Kurokawa, T. *et al*., *Biotechnology* 7:1163 (1989)).

### Summary of the Invention

This invention relates to the discovery that multivalent forms of single-chain antigen-binding proteins have significant utility beyond that of the monovalent single-chain antigen-binding proteins. A multivalent antigen-binding protein has more than one antigen-binding site. Enhanced binding activity, di-and multi-specific binding, and other novel uses of multivalent antigen-binding proteins have been demonstrated or are envisioned.

The present invention provides a method of producing a multivalent antigen-binding protein characterised in that it comprises:
(a) producing a composition comprising single-chain molecules, each single-chain molecule comprising:
   (i) a first polypeptide comprising the binding portion of the variable region of an antibody heavy or light chain;
   (ii) a second polypeptide comprising the binding portion of the variable region of an antibody heavy or light chain;
      and
   (iii) a peptide linker linking the said first and second polypeptides (i) and (ii) into the said single-chain molecule;
(b) dissociating the said single-chain molecules;
(c) re-associating the said single-chain molecules;
(d) separating multivalent antigen-binding proteins from the said single-chain molecules;
   and
(e) recovering the said multivalent proteins.

The dissociation may be caused by dialysis against a dissociating solution. The reassociation may be caused by dialysis against a refolding solution or a refolding agent. The single-chain molecules may be concentrated before separating the multivalent protein from the single-chain molecules. For example, the concentrating may occur from approximately 0.5 mg/ml single-chain molecule to the concentration at which precipitation starts.

In one preferred embodiment, a variable light chain of a first single-chain antigen-binding protein associates with a variable heavy chain of a second single-chain antigen-binding protein.

Having indicated the scope of the present invention, it may now be further described and illustrated in more general terms.

An advantage of using multivalent antigen-binding proteins instead of single-chain antigen-binding molecules or Fab fragments lies in the enhanced binding ability of the multivalent form. Enhanced binding occurs because the multivalent form has more binding sites per molecule. Another advantage is the ability to use multivalent antigen-binding proteins as multi-specific binding molecules.

An advantage of using multivalent antigen-binding proteins instead of whole antibodies, is the enhanced clearing of the multivalent antigen-binding proteins from the serum due to their smaller size as compared to whole antibodies which may afford lower background in imaging applications. Multivalent antigen-binding proteins may penetrate solid tumors better than monoclonals, resulting in better tumor-fighting ability. Also, because they are smaller and lack the Fc component of intact antibodies, the multivalent antigen-binding proteins which may be produced according to the present invention may be less immunogenic than whole antibodies. The Fc component of whole antibodies also contains binding sites for liver, spleen and certain other cells and its absence should thus reduce accumulation in non-target tissues.

Another advantage of multivalent antigen-binding proteins is the ease with which they may be produced and engineered, as compared to the myeloma-fusing technique pioneered by Kohler and Milstein that is used to produce whole antibodies.

### Brief Description of the Drawings.

The present invention as defined in the claims can be better understood with reference to the text and to the following drawings:
FIG. **1A** is a schematic two-dimensional representation of two identical single-chain antigen-binding protein molecules, each comprising a variable light chain region (V_{L}), a variable heavy chain region (V_{H}), and a polypeptide linker joining the two regions. The single-chain antigen-binding protein molecules are shown binding antigen in their antigen-binding sites.
FIG. **1B** depicts a hypothetical homodivalent antigen-binding protein formed by association of the polypeptide linkers of two monovalent single-chain antigen-binding proteins from Fig. 1A (the Association model). The divalent antigen-binding protein is formed by the concentration-driven association of two identical single-chain antigen-binding protein molecules.
FIG. **1C** depicts the hypothetical divalent protein of FIG. 1B with bound antigen molecules occupying both antigen-binding sites.
FIG. **2A** depicts the hypothetical homodivalent protein of Figure 1B.
FIG. **2B** depicts three single-chain antigen-binding protein molecules associated in a hypothetical trimer.
FIG. **2C** depicts a hypothetical tetramer of four single-chain antigen-binding protein molecules.
FIG. **3A** depicts two separate and distinct monovalent single-chain antigen-binding proteins, Anti-A single-chain antigen-binding protein and Anti-B single-chain antigen-binding protein, with different antigen specificities, each individually binding either Antigen A or Antigen B.
FIG. **3B** depicts a hypothetical bispecific heterodivalent antigen-binding protein formed from the single-chain antigen-binding proteins of Fig. 3A according to the Association model.
FIG. **3C** depicts the hypothetical heterodivalent antigen-binding protein of FIG. **3B** binding bispecifically, i.e., binding the two different antigens, A and B.
FIG. **4A** depicts two identical single-chain antigen-binding protein molecules, each having a variable light chain region (V_{L}), a variable heavy chain region (V_{H}), and a polypeptide linker joining the two regions. The single-chain antigen-binding protein molecules are shown binding identical antigen molecules in their antigen-binding sites.
FIG. **4B** depicts a hypothetical homodivalent protein formed by the rearrangement of the V_{L} and V_{H} regions shown in FIG. 4A (the Rearrangement model). Also shown is bound antigen.
FIG. **5A** depicts two single-chain protein molecules, the first having an anti-B V_{L} and an anti-A V_{H}, and the second having an anti-A V_{L} and an anti-B V_{H}. The figure shows the non-complementary nature of the V_{L} and V_{H} regions in each single-chain protein molecule.
FIG. **5B** shows a hypothetical bispecific heterodivalent antigen-binding protein formed by rearrangement of the two single-chain proteins of Figure 5A.
FIG. **5C** depicts the hypothetical heterodivalent antigen-binding protein of FIG. 5B with different antigens A and B occupying their respective antigen-binding sites.
FIG. **6A** is a schematic depiction of a hypothetical trivalent antigen-binding protein according to the Rearrangement model.
FIG. **6B** is a schematic depiction of a hypothetical tetravalent antigen-binding protein according to the Rearrangement model.
FIG. **7** is a chromatogram depicting the separation of CC49/212 antigen-binding protein monomer from dimer on a cation exchange high performance liquid chromatographic column. The column is a PolyCAT A aspartic acid column (Poly WC, Columbia, MD). Monomer is shown as Peak 1, eluting at 27.32 min., and dimer is shown as Peak 2, eluting at 55.52 min.
FIG. **8** is a chromatogram of the purified monomer from Fig. 7. Monomer elutes at 21.94 min., preceded by dimer (20.135 min.) and trimer (18.640 min.). Gel filtration column, Protein-Pak 300SW (Waters Associates, Milford, MA).
FIG. **9** is a similar chromatogram of purified dimer (20.14 min.) from Fig. 7, run on the gel filtration HPLC column of Fig. 8.
FIG. **10A** is an amino acid (SEQ ID NO. 11) and nucleotide (SEQ ID NO. 10) sequence of the single-chain protein comprising the 4-4-20 V_{L} region connected through the 212 linker polypeptide to the CC49 V_{H} region.
FIG. **10B** is an amino acid (SEQ ID NO. 13) and nucleotide (SEQ ID NO. 12) sequence of the single-chain protein comprising the CC49 V_{L} region connected through the 212 linker polypeptide to the 4-4-20 V_{H} region.
FIG. **11** is a chromatogram depicting the separation of the monomer (27.83 min.) and dimer (50.47 min.) forms of the CC49/212 antigen-binding protein by cation exchange, on a PolyCAT A cation exchange column (Poly LC, Columbia, MD).
Fig. **12** shows the separation of monomer (17.65 min.), dimer (15.79 min.), trimer (14.19 min.), and higher oligomers (shoulder at about 13.09 min.) of the B6.2/212 antigen-binding protein. This separation depicts the results of a 24-hour treatment of a 1.0 mg/ml B6.2/212 single-chain antigen-binding protein sample. A TSK G2000SW gel filtration HPLC column was used, Toyo Soda, Tokyo, Japan.
Fig. **13** shows the results of a 24-hour treatment of a 4.0 mg/ml CC49/212 antigen-binding protein sample, generating monomer, dimer, and trimer at 16.91, 14.9, and 13.42 min., respectively. The same TSK gel filtration column was used as in Fig. 12.
Fig. **14** shows a schematic view of the four-chain structure of a human IgG molecule.
Fig. **15A** is an amino acid (SEQ ID NO. 15) and nucleotide (SEQ ID NO. 14) sequence of the 4-4-20/212 single-chain antigen-binding protein with a single cysteine hinge.
Fig. **15B** is an amino acid (SEQ ID NO. 17) and nucleotide (SEQ. ID NO. 16) sequence of the 4-4-20/212 single-chain antigen-binding protein with the two-cysteine hinge.
Fig. **16** shows the amino acid (SEQ ID NO. 19) and nucleotide (SEQ ID NO. 18) sequence of a divalent CC49/212 single-chain antigen-binding protein.
Fig. **17** shows the expression of the divalent CC49/212 single-chain antigen-binding protein of Fig. 16 at 42°C, on an SDS-PAGE gel containing total *E*. *coli* protein. Lane 1 contains the molecular weight standards. Lane 2 is the uninduced *E. coli* production strain grown at 30°C. Lane 3 is divalent CC49/212 single-chain antigen-binding protein induced by growth at 42°C. The arrow shows the band of expressed divalent CC49/212 single-chain antigen-binding protein.
Fig. **18** is a graphical representation of four competition radioimmunoassays (RIA) in which unlabeled CC49 IgG (open circles) CC49/212 single-chain antigen-binding protein (closed circles) and CC49/212 divalent antigen-binding protein (closed squares) and anti-fluorescein 4-4-20/212 single-chain antigen-binding protein (open squares) competed against a CC49 IgG radiolabeled with ¹²⁵I for binding to the TAG-72 antigen on a human breast carcinoma extract.
Figure **19A** is an amino acid (SEQ ID NO. 21) and nucleotide (SEQ ID NO. 20) sequence of the single-chain polypeptide comprising the 4-4-20 V_{L} region connected through the 217 linker polypeptide to the CC49 V_{H} region.
Figure **19B** is an amino acid (SEQ ID NO. 23) and nucleotide (SEQ ID NO. 22) sequence of the single-chain polypeptide comprising the CC49 V_{L} region connected through the 217 linker polypeptide to the 4-4-20 V_{H} region.
Figure **20** is a chromatogram depicting the purification of CC49/4-4-20 heterodimer Fv on a cation exchange high performance liquid chromatographic column. The column is a PolyCAT A aspartic acid column (Poly LC, Columbia, MD). The heterodimer Fv is shown as peak 5, eluting at 30.10 min.
Figure **21** is a coomassie-blue stained 4-20% SDS-PAGE gel showing the proteins separated in Figure 20. Lane 1 contains the molecular weight standards. Lane 3 contains the starting material before separation. Lanes 4-8 contain fractions 2, 3, 5, 6 and 7 respectively. Lane 9 contains purified CC49/212.
Figure **22A** is a chromatogram used to determine the molecular size of fraction 2 from Figure **20.** A TSK G3000SW gel filtration HPLC column was used (Toyo Soda, Tokyo, Japan).
Figure **22B** is a chromatogram used to determine the molecular size of fraction 5 from Figure **20.** A TSK G3000SW gel filtration HPLC column was used (Toyo Soda, Tokyo, Japan).
Figure **22C** is a chromatogram used to determine the molecular size of fraction 6 from Figure **20.** A TSK G30005W gel filtration HPLC column was used (Toyo Soda, Tokyo, Japan).
Figure **23** shows a Scatchard analysis of the fluorescein binding affinity of the CC49 4-4-20 heterodimer Fv (fraction 5 in Figure **20**).
Figure **24** is a graphical representation of three competition enzyme-linked immunosorbent assays (ELISA) in which unlabeled CC49 4-4-20 Fv (closed squares) CC49/212 single-chain Fv (open squares) and MOPC-21 IgG (+) competed against a biotin-labeled CC49 IgG for binding to the TAG-72 antigen on a human breast carcinoma extract. MOPC-21 is a control antibody that does not bind to TAG-72 antigen.
Figure **25** shows a coomassie-blue stained non-reducing 4-20% SDS-PAGE gel. Lanes 1 and 9 contain the molecular weight standards. Lane 3 contains the 4-4-20/212 CPPC single-chain antigen-binding protein after purification. Lane 4, 5 and 6 contain the 4-4-20/212 CPPC single-chain antigen-binding protein after treatment with DTT and air oxidation. Lane 7 contains 4-4-20/212 single-chain antigen-binding protein.
Figure **26** shows a coomassie-blue stained reducing 4-20% SDS-PAGE gel (samples were treated with β-mercaptoethanol prior to being loaded on the gel). Lanes 1 and 8 contain the molecular weight standards. Lane 3 contains the 4-4-20/212 CPPC single-chain antigen-binding protein after treatment with *bis*-maleimidehexane. Lane 5 contains peak 1 of *bis*-maleimidehexane treated 4-4-20/212 CPPC single-chain antigen-binding protein. Lane 6 contains peak 3 of *bis*-maleimidehexane treated 4-4-20/212 CPPC single-chain antigen-binding protein.

### Detailed Description of the Preferred Embodiments

This invention relates to the discovery that multivalent forms of single-chain antigen-binding proteins have significant utility beyond that of the monovalent single-chain antigen-binding proteins. A multivalent antigen-binding protein has more than one antigen-binding site. For the purposes of this application, "valent" refers to the numerosity of antigen binding sites. Thus, a bivalent protein refers to a protein with two binding sites. Enhanced binding activity, bi- and multi-specific binding, and other novel uses of multivalent antigen-binding proteins have been demonstrated or are envisioned. The present invention is directed to the above method of producing a multivalent antigen-binding protein.

For the present purposes, the term "multivalent" means any assemblage, covalently or non-covalently joined, of two or more single-chain proteins, the assemblage having more than one antigen-binding site. The single-chain proteins composing the assemblage may have antigen-binding activity, or they may lack antigen-binding activity individually but be capable of assembly into active multivalent antigen-binding proteins. The term "multivalent" encompasses bivalent, trivalent, tetravalent, etc. It is envisioned that multivalent forms above bivalent may be useful for certain applications.

A preferred form of the multivalent antigen-binding protein comprises bivalent proteins, including heterobivalent and homobivalent forms. The term "bivalent" means an assemblage of single-chain proteins associated with each other to form two antigen-binding sites. The term "heterobivalent" indicates multivalent antigen-binding proteins that are bispecific molecules capable of binding to two different antigenic determinants. Therefore, heterobivalent proteins have two antigen-binding sites that have different binding specificities. The term "homobivalent" indicates that the two binding sites are for the same antigenic determinant.

The terms "single-chain molecule" or "single-chain protein" are used interchangeably here. They are structurally defined as comprising the binding portion of a first polypeptide from the variable region of an antibody, associated with the binding portion of a second polypeptide from the variable region of an antibody, the two polypeptides being joined by a peptide linker linking the first and second polypeptides into a single polypeptide chain. The single polypeptide chain thus comprises a pair of variable regions connected by a polypeptide linker. The regions may associate to form a functional antigen-binding site, as in the case wherein the regions comprise a light-chain and a heavy-chain variable region pair with appropriately paired complementarity determining regions (CDRs). In this case, the single-chain protein is referred to as a "single-chain antigen-binding protein" or "single-chain antigen-binding molecule."

Alternatively, the variable regions may have unnaturally paired CDRs or may both be derived from the same kind of antibody chain, either heavy or light, in which case the resulting single-chain molecule may not display a functional antigen-binding site. The single-chain antigen-binding protein molecule is more fully described in U.S. Patent No. 4,946,778 (Ladner *et al*.).

Without being bound by any particular theory, the inventors speculate on several models which can equally explain the phenomenon of multivalence. The inventors' models are presented herein for the purposes of illustration only. The invention is useful and operable regardless of the precise mechanism of multivalence.

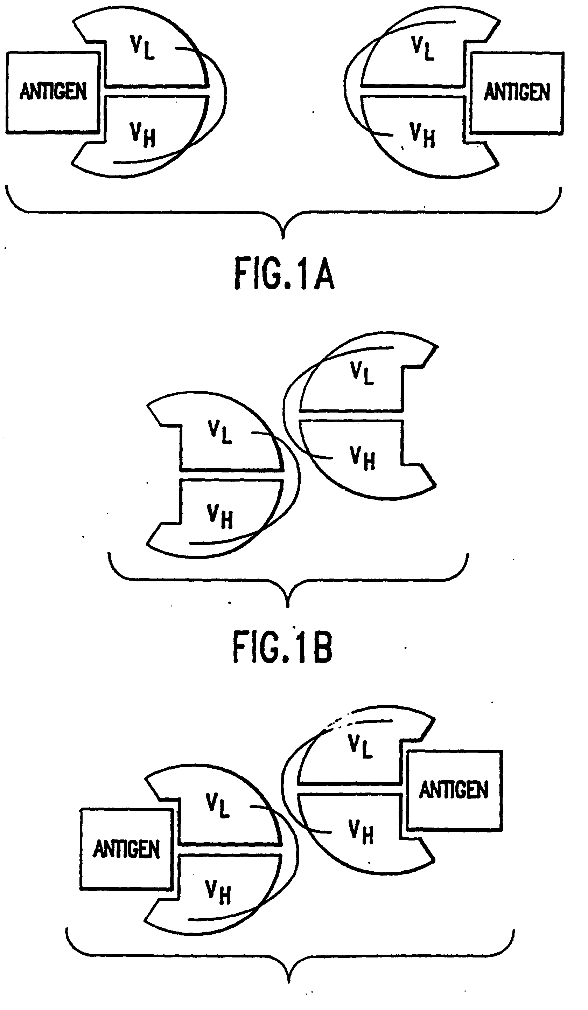

Figure 1 depicts the first hypothetical model for the creation of a multivalent protein, the "Association" model Fig. 1A shows two monovalent single-chain antigen-binding proteins, each composed of a V_{L}, a V_{H}, and a linker polypeptide covalently bridging the two. Each monovalent single-chain antigen-binding protein is depicted having an identical antigen-binding site containing antigen. Figure 1B shows the simple association of the two single-chain antigen-binding proteins to create the bivalent form of the multivalent protein. It is hypothesized that simple hydrophobic forces between the monovalent proteins are responsible for their association in this manner. The origin of the multivalent proteins may be traceable to their concentration dependence. The monovalent units retain their original association between the V_{H} and V_{L} regions. Figure 1C shows the newly-formed homobivalent protein binding two identical antigen molecules simultaneously. Homobivalent antigen-binding proteins are necessarily monospecific for antigen.

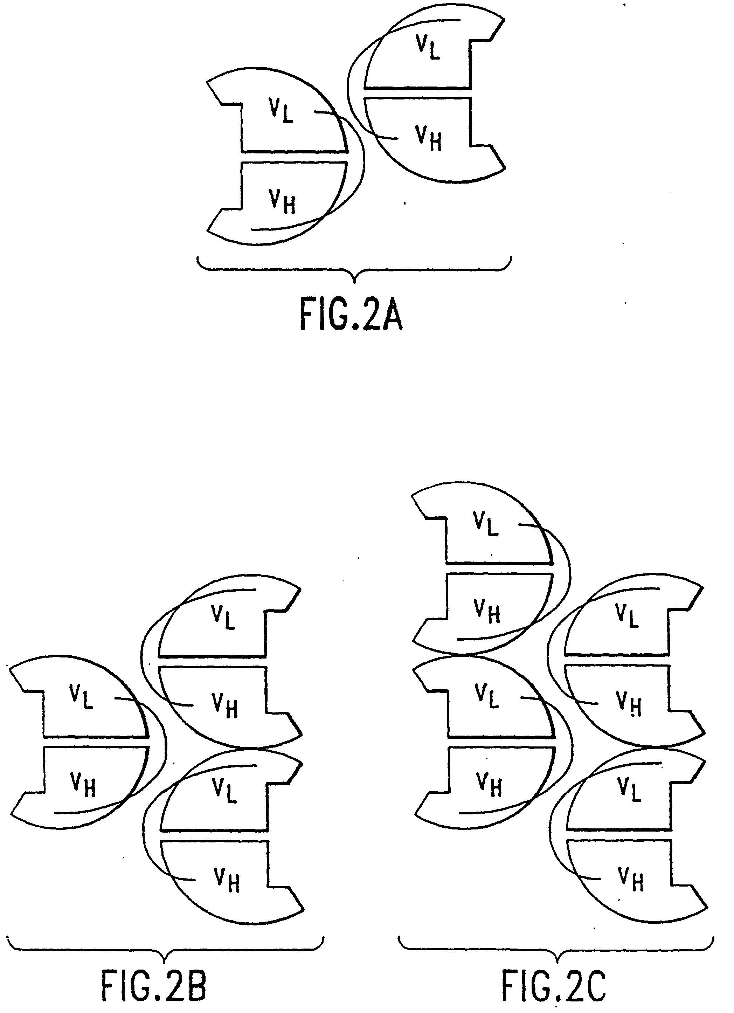

Homovalent proteins are depicted in Figs. 2A through 2C formed according to the Association model. Fig. 1A depicts a homobivalent protein, Fig. 2B a trivalent protein, and Fig. 2C a tetravalent protein. Of course, the limitations of two-dimensional images of three-dimensional objects must be taken into account. Thus, the actual spatial arrangement of multivalent proteins can be expected to vary somewhat from these figures.

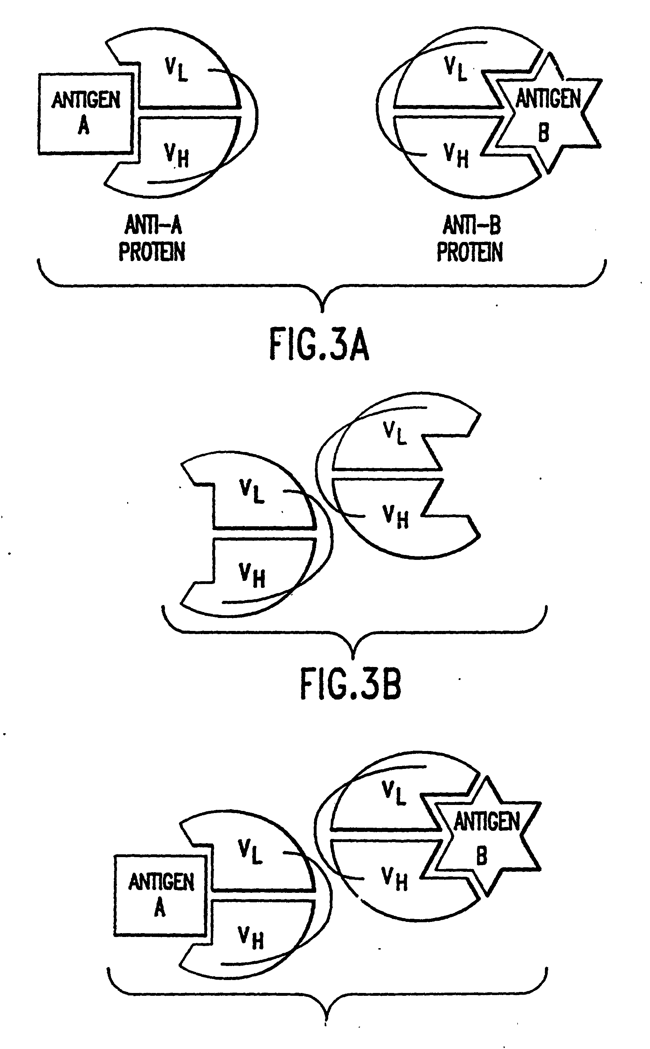

A heterobivalent antigen-binding protein has two different binding sites, the sites having different binding specificities. Figures 3A through C depict the Association model pathway to the creation of a heterobivalent protein. Figure 3A shows two monovalent single-chain antigen-binding proteins, Anti-A single-chain antigen-binding protein and Anti-B single-chain antigen-binding protein, with antigen types A and B occupying the respective binding sites. Figure 3B depicts the heterobivalent protein formed by the simple association of the original monovalent proteins. Figure 3C shows the heterobivalent protein having bound antigens A and B into the antigen-binding sites. Figure 3C therefore shows the heterobivalent protein binding in a bispecific manner.

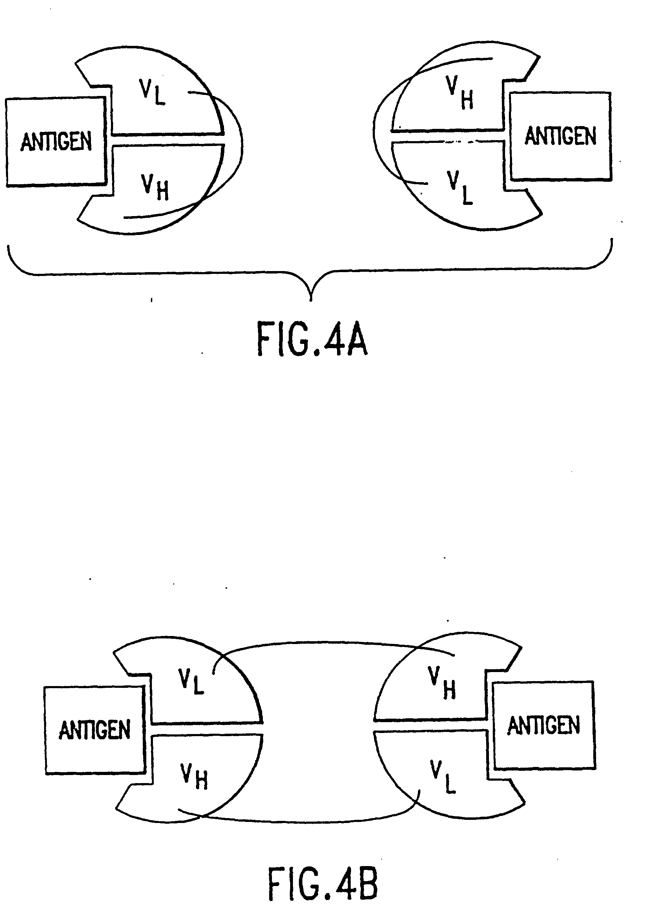

An alternative model for the formation of multivalent antigen-binding proteins is shown in Figures 4 through 6. This "Rearrangement" model hypothesizes the dissociation of the variable region interface by contact with dissociating agents such as guanidine hydrochloride, urea, or alcohols such as ethanol, either alone or in combination. Combinations and relevant concentration ranges of dissociating agents are recited in the discussion concerning dissociating agents, and in Example 2. Subsequent re-association of dissociated regions allows variable region recombination differing from the starting single-chain proteins, as depicted in Fig. 4B. The homobivalent antigen-binding protein of Figure 4B is formed from the parent single-chain antigen-binding proteins shown in Figure 4A, the recombined bivalent protein having V_{L} and V_{H} from the parent monovalent single-chain proteins. The homobivalent protein of Figure 4B is a fully functional monospecific bivalent protein, shown actively binding two antigen molecules.

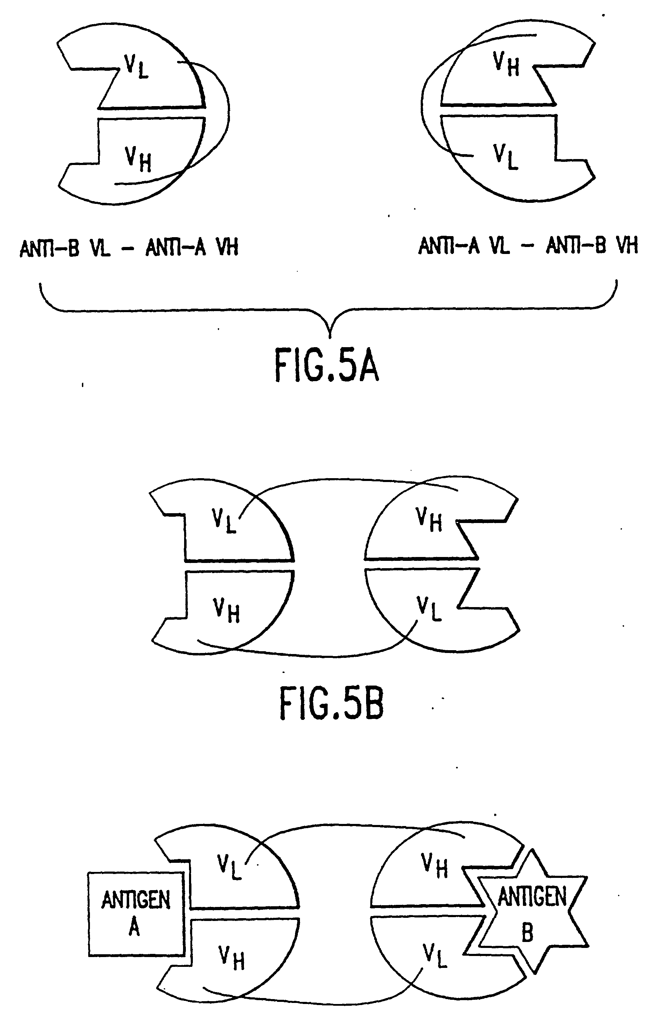

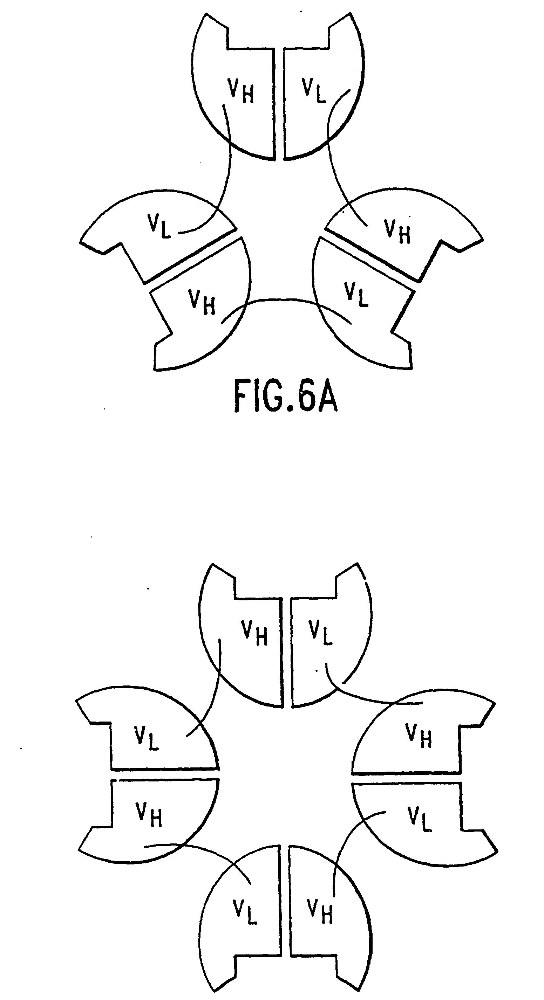

Figures 5A-5C show the formation of heterobivalent antigen-binding proteins via the Rearrangement model. Figure 5A shows a pair of single-chain proteins, each having a V_{L} with complementarity determining regions (CDRs) that do not match those of the associated V_{H}. These single-chain proteins have reduced or no ability to bind antigen because of the mixed nature of their antigen-binding sites, and thus are made specifically to be assembled into multivalent proteins through this route. Figure 5B shows the heterobivalent antigen-binding protein formed whereby the V_{H} and V_{L} regions of the parent proteins are shared between the separate halves of the heterobivalent protein. Figure 5C shows the binding of two different antigen molecules to the resultant functional bispecific heterobivalent protein. The Rearrangement model also explains the generation of multivalent proteins of a higher order than bivalent, as it can be appreciated that more than a pair of single-chain proteins can be reassembled in this manner. These are depicted in Figures 6A and 6B.

One of the major utilities of the multivalent antigen-binding protein is in the heterobivalent form, in which one specificity is for one type of hapten or antigen, and the second specificity is for a second type of hapten or antigen. A multivalent molecule having two distinct binding specificities has many potential uses. For instance, one antigen binding site may be specific for a cell-surface epitope of a target cell, such as a tumor cell or other undesirable cell. The other antigen-binding site may be specific for a cell-surface epitope of an effector cell, such as the CD3 protein of a cytotoxic T-cell. In this way, the heterobivalent antigen-binding protein may guide a cytotoxic cell to a particular class of cells that are to be preferentially attacked.

Other uses of heterobivalent antigen-binding proteins are the specific targeting and destruction of blood clots by a bispecific molecule with specificity for tissue plasminogen activator (tPA) and fibrin; the specific targeting of prodrug activating enzymes to tumor cells by a bispecific molecule with specificity for tumor cells and enzyme; and specific targeting of cytotoxic proteins to tumor cells by a bispecific molecule with specificity for tumor cells and a cytotoxic protein. This list is illustrative only.

Multivalent antigen-binding proteins may also be used in purification and biosensors. Affinity purification is made possible by affixing the multivalent antigen-binding protein to a support, with the antigen-binding sites exposed to and in contact with the ligand molecule to be separated, and thus purified. Biosensors generate a detectable signal upon binding a specific antigen to an antigen-binding molecule, with subsequent processing of the signal. Multivalent antigen-binding proteins, when used as the antigen-binding molecule in biosensors, may change conformation upon binding, thus generating a signal that may be detected.

Essentially all of the uses for which monoclonal or polyclonal antibodies, or fragments thereof, have been envisioned by the prior art, can be addressed by the multivalent proteins which may be produced according to the present invention. These uses include detectably-labelled forms of the multivalent protein. Types of labels are well-known to those of ordinary skill in the art. They include radiolabelling, chemiluminescent labelling, fluorochromic labelling, and chromophoric labelling. Other uses include imaging the internal structure of an animal (including a human) by administering an effective amount of a labelled form of the multivalent protein and measuring detectable radiation associated with the animal. They also include improved immunoassays, including sandwich immunoassay, competitive immunoassay, and other immunoassays wherein the labelled antibody can be replaced by the multivalent antigen-binding protein.

One method of producing multivalent antigen-binding proteins involves separating the multivalent proteins from a production composition that comprises both multivalent and single-chain proteins, as represented in Example 1. The method comprises producing a composition of multivalent and single-chain proteins, separating the multivalent proteins from the single-chain proteins, and recovering the multivalent proteins.

More particularly, a preferred method of producing multivalent antigen-binding proteins according to the present invention comprises the steps of producing single-chain protein molecules, dissociating said single-chain molecules, reassociating the single-chain molecules such that a significant fraction of the resulting composition includes multivalent forms of the single-chain antigen-binding proteins, separating multivalent antigen-binding proteins from single-chain molecules, and recovering the multivalent proteins. This process is illustrated with more detail in Example 2. For the purposes of this method, the term "producing a composition comprising single-chain molecules" may indicate the actual production of these molecules. The term may also include procuring them from whatever commercial or institutional source makes them available. Use of the term "producing single-chain proteins" means production of single-chain proteins by any process, but preferably according to the process set forth in U.S. Patent No. 4,946,778 (Ladner *et al*.). Briefly, that patent pertains to a single polypeptide chain antigen-binding molecule which has binding specificity and affinity substantially similar to the binding specificity and affinity of the aggregate light and heavy chain variable regions of an antibody, to genetic sequences coding therefore, and to recombinant DNA methods of producing such molecules, and uses for such molecules. The single-chain protein produced by the Ladner *et al*. methodology comprises two regions linked by a linker polypeptide. The two regions are termed the V_{H} and V_{L} regions, each region comprising one half of a functional antigen-binding site.

The term "dissociating said single-chain molecules" means to cause the physical separation of the two variable regions of the single-chain protein without causing denaturation of the variable regions.

"Dissociating agents" are defined herein to include all agents capable of dissociating the variable regions, as defined above. In the context of this invention, the term includes the well-known agents alcohol (including ethanol), guanidine hydrochloride (GuHCl), and urea. Others will be apparent to those of ordinary skill in the art, including detergents and similar agents capable of interrupting the interactions that maintain protein conformation. In the preferred embodiment, a combination of GuHCl and ethanol (EtOH) is used as the dissociating agent. A preferred range for ethanol and GuHCl is from 0 to 50% EtOH, vol/vol, 0 to 2.0 moles per liter (M) GuHCl. A more preferred range is from 10-30% EtOH and 0.5-1.0 M GuHCl, and a most preferred range is 20% EtOH, 0.5 M GuHCl. A preferred dissociation buffer contains 0.5 M guanidine hydrochloride, 20% ethanol, 0.05 M TRIS, and 0.01 M CaCl₂, pH 8.0.

Use of the term "re-associating said single-chain molecules" is meant to describe the reassociation of the variable regions by contacting them with a buffer solution that allows reassociation. Such a buffer is preferably used in the present invention and is characterized as being composed of 0.04 M MOPS, 0.10 M calcium acetate, pH 7.5. Other buffers allowing the reassociation of the V_{L} and V_{H} regions are well within the expertise of one of ordinary skill in the art.

The separation of the multivalent protein from the single-chain molecules occurs by use of standard techniques known in the art, particularly including cation exchange or gel filtration chromatography.

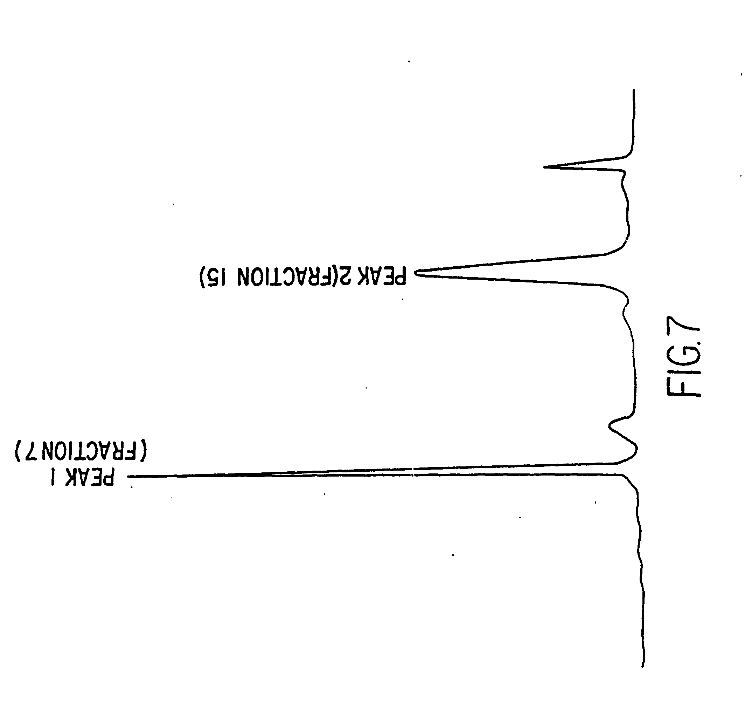

Cation exchange chromatography is the general liquid chromatographic technique of ion-exchange chromatography utilizing anion columns well-known to those of ordinary skill in the art. In this invention, the cations exchanged are the single-chain and multivalent protein molecules. Since multivalent proteins will have some multiple of the net charge of the single-chain molecule, the multivalent proteins are retained more strongly and are thus separated from the single-chain molecules. The preferred cationic exchanger of the present invention is a polyaspartic acid column, as shown in Figure 7. Figure 7 depicts the separation of single-chain protein (Peak 1, 27.32 min.) from bivalent protein (Peak 2, 55.54 min.) Those of ordinary skill in the art will realize that the invention is not limited to any particular type of chromatography column, so long as it is capable of separating the two forms of protein molecules.

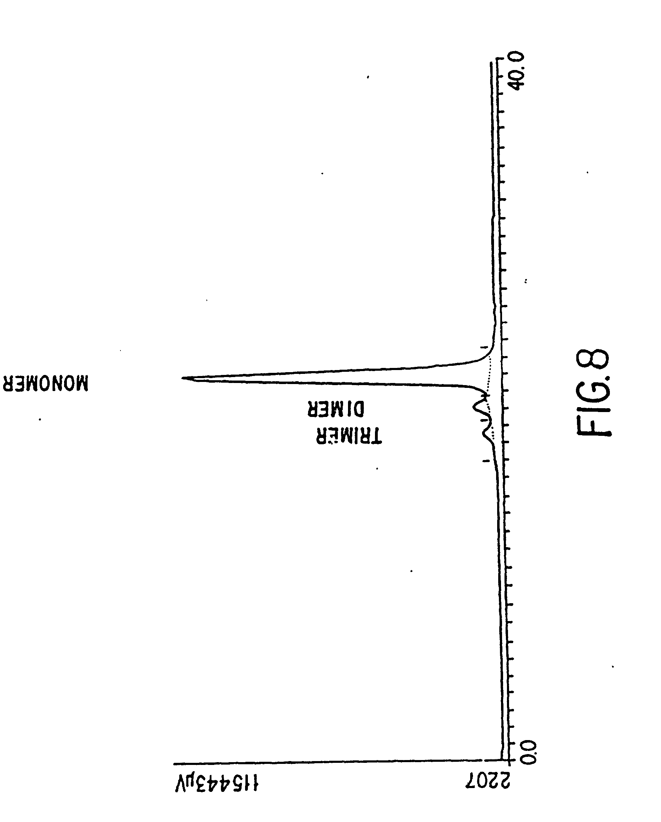

Gel filtration chromatography is the use of a gel-like material to separate proteins on the basis of their molecular weight. A "gel" is a matrix of water and a polymer, such as agarose or polymerized acrylamide. The present invention encompasses the use of gel filtration HPLC (high performance liquid chromatography), as will be appreciated by one of ordinary skill in the art. Figure 8 is a chromatogram depicting the use of a Waters Associates' Protein-Pak 300 SW gel filtration column to separate monovalent single-chain protein from multivalent protein, including the monomer (21.940 min.), bivalent protein (20.135 min.), and trivalent protein (18.640 min.).

Recovering the multivalent antigen-binding proteins is accomplished by standard collection procedures well known in the chemical and biochemical arts. In the context of the present invention recovering the multivalent protein preferably comprises collection of eluate fractions containing the peak of interest from either the cation exchange column, or the gel filtration HPLC column. Manual and automated fraction collection are well-known to one of ordinary skill in the art. Subsequent processing may involve lyophilization of the eluate to produce a stable solid, or further purification.

When producing multivalent antigen-binding proteins one may also start with purified single-chain proteins at a lower concentration, and then increase the concentration until some significant fraction of multivalent proteins is formed. The multivalent proteins are then separated and recovered. The concentrations conducive to formation of multivalent proteins in this manner are from about 0.5 milligram per milliliter (mg/ml) to the concentration at which precipitates begin to form.

The use of the term "substantially free" when used to describe a composition of multivalent and single-chain antigen-binding protein molecules means the lack of a significant peak corresponding to the single-chain molecule, when the composition is analyzed by cation exchange chromatography, as disclosed in Example 1 or by gel filtration chromatography as disclosed in Example 2.

By use of the term "aqueous composition" is meant any composition of single-chain molecules and multivalent proteins including a portion of water. In the same context, the phrase "an excess of multivalent antigen-binding protein over single-chain molecules" indicates that the composition comprises more than 50% of multivalent antigen-binding protein.

The use of the term "cross-linking" refers to chemical means by which one can produce multivalent antigen-binding proteins from monovalent single-chain protein molecules. For example, the incorporation of a cross-linkable sulfhydryl chemical group as a cysteine residue in the single-chain proteins allows cross-linking by mild reduction of the sulfhydryl group. Both monospecific and multispecific multivalent proteins can be produced from single-chain proteins by cross-linking the free cysteine groups from two or more single-chain proteins, causing a covalent chemical linkage to form between the individual proteins. Free cysteines have been engineered into the C-terminal portion of the 4-4-20/212 single-chain antigen-binding protein, as discussed in Example 5 and Example 8. These free cysteines may then be cross-linked to form multivalent antigen-binding proteins.

Single-chain proteins may be produced comprising: (a) a first polypeptide comprising the binding portion of the variable region of an antibody light chain; (b) a second polypeptide comprising the binding portion of the variable region of an antibody light chain; and (c) a peptide linker linking said first and second polypeptides (a) and (b) into said single-chain protein. A similar single-chain protein comprising the heavy chain variable regions is also possible. Genetic sequences encoding these molecules are also contemplated. Since these proteins are comprised of two similar variable regions, they do not necessarily have any antigen-binding capability.

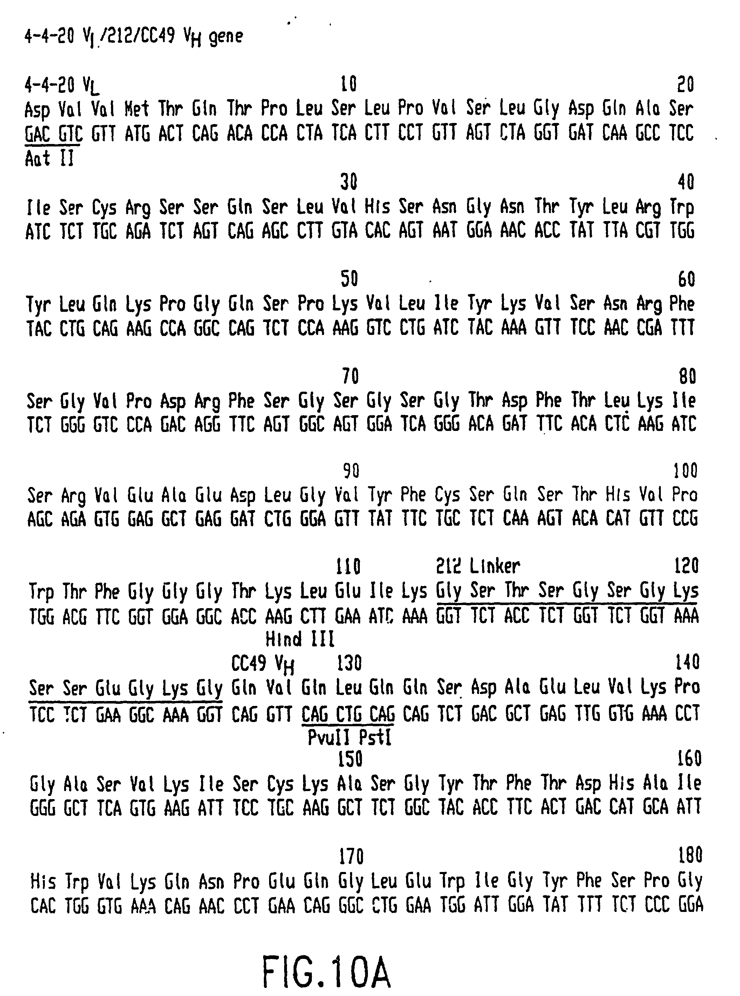

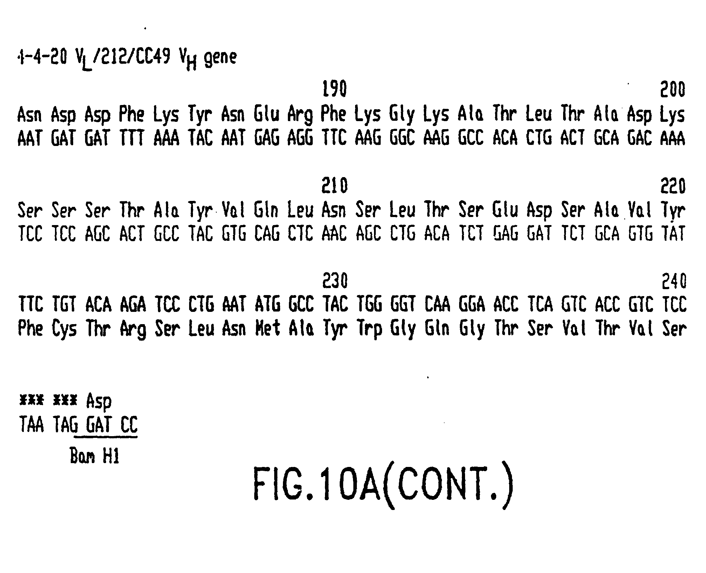

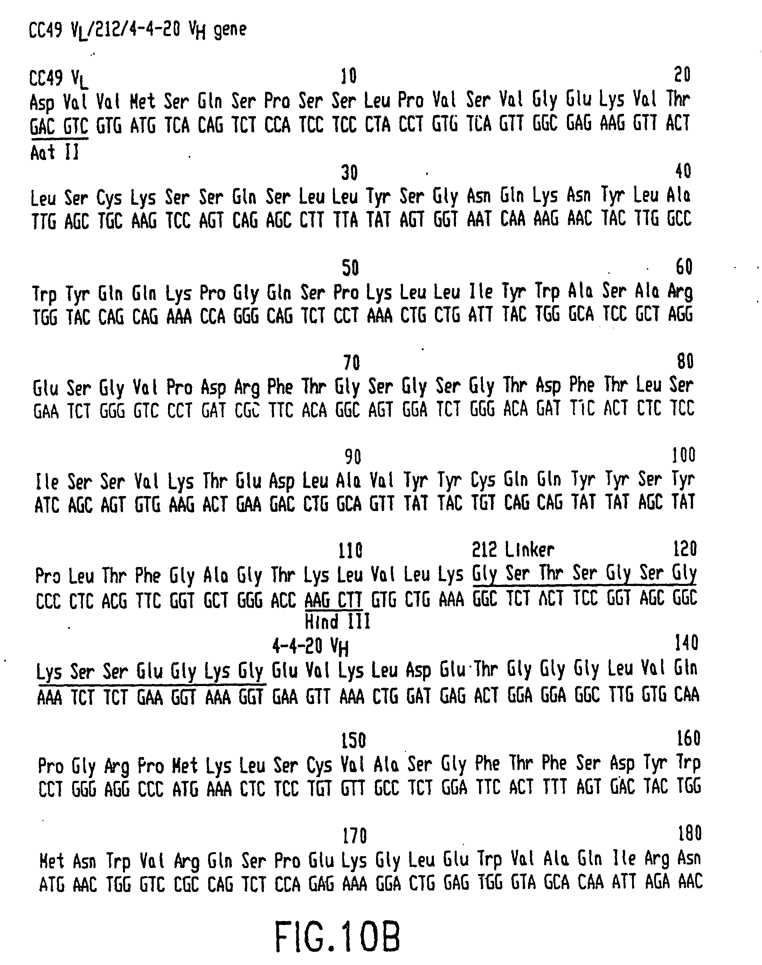

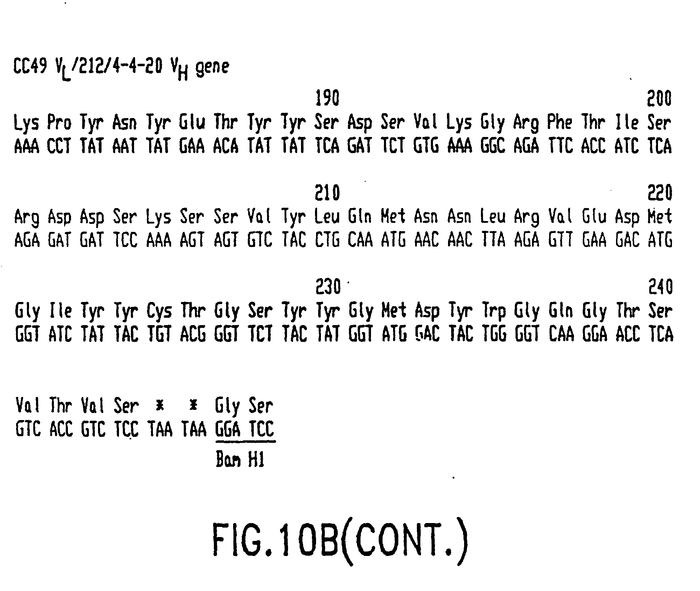

There is also possible a DNA sequence encoding a bispecific bivalent antigen-binding protein. Example 4 and Example 7 discuss in detail the sequences that appear in Figs. 10A and 10B that allow one of ordinary skill to construct a heterobivalent antigen-binding molecule. Figure 10A is an amino acid and nucleotide sequence listing of the single-chain protein comprising the 4-4-20 V_{L} region connected through the 212 linker polypeptide to the CC49 V_{H} region. Figure 10B is a similar listing of the single-chain protein comprising the CC49 V_{L} region connected through the 212 linker polypeptide to the 4-4-20 V_{H} region. Subjecting a composition including these single-chain molecules to dissociating and subsequent re-associating conditions results in the production of a bivalent protein with two different binding specificities.

Synthesis of DNA sequences is well known in the art, and possible through at least two routes. First, it is well-known that DNA sequences may be synthesized through the use of automated DNA synthesizers *de novo*, once the primary sequence information is known. Alternatively, it is possible to obtain a DNA sequence coding for a multivalent single-chain antigen-binding protein by removing the stop codons from the end of a gene encoding a single-chain antigen-binding protein, and then inserting a linker and a gene encoding a second single-chain antigen-binding protein. Example 6 demonstrates the construction of a DNA sequence coding for a bivalent single-chain antigen-binding protein. Other methods of genetically constructing multivalent single-chain antigen-binding proteins are also contemplated.

Having now generally described this invention the same will better be understood by reference to certain specific examples which are included for purposes of illustration.

### Example 1

### Production of Multivalent Antigen-Binding Proteins During Purification

In the production of multivalent antigen-binding proteins, the same recombinant *E. coli* production system that was used for prior single-chain antigen-binding protein production was used. See Bird, *et al*., *Science* 242:423 (1988). This production system produced between 2 and 20% of the total *E. coli* protein as antigen-binding protein. For protein recovery, the frozen cell paste from three 10-liter fermentations (600-900 g) was thawed overnight at 4 °C and gently resuspended at 4 °C in 50 mM Tris-HCl, 1.0 mM EDTA, 100 mM KCI, 0.1 mM PMSF, pH 8.0 (lysis buffer), using 10 liters of lysis buffer for every kilogram of wet cell paste. When thoroughly resuspended, the chilled mixture was passed three times through a Manton-Gaulin cell homogenizer to totally lyse the cells. Because the cell homogenizer raised the temperature of the cell lysate to 25 ±5°C, the cell lysate was cooled to 5 ±2°C with a Lauda/Brinkman chilling coil after each pass. Complete lysis was verified by visual inspection under a microscope.

The cell lysate was centrifuged at 24,300 g for 30 min. at 6 °C using a Sorvall RC-5B centrifuge. The pellet containing the insoluble antigen-binding protein was retained, and the supernatant was discarded. The pellet was washed by gently scraping it from the centrifuge bottles and resuspending it in 5 liters of lysis buffer/kg of wet cell paste. The resulting 3.0- to 4.5-liter suspension was again centrifuged at 24,300 g for 30 min. at 6 °C, and the supernatant was discarded. This washing of the pellet removes soluble *E*. *coli* proteins and can be repeated as many as five times. At any time during this washing procedure the material can be stored as a frozen pellet at -20°C. A substantial time saving in the washing steps can be accomplished by utilizing a Pellicon tangential flow apparatus equipped with 0.22-µm microporous filters, in place of centrifugation.

The washed pellet was solubilized at 4°C in freshly prepared 6 M guanidine hydrochloride, 50 mM Tris-HCl, 10 mM CaCl₂, 50 mM KCl, pH 8.0 (dissociating buffer), using 9 ml/g of pellet. If necessary, a few quick pulses from a Heat Systems Ultrasonics tissue homogenizer can be used to complete the solubilization. The resulting suspension was centrifuged at 24,300g for 45 min at 6°C and the pellet was discarded. The optical density of the supernatant was determined at 280 nm and if the OD₂₈₀ was above 30, additional dissociating buffer was added to obtain an OD₂₈₀ of approximately 25.

The supernatant was slowly diluted into cold (4-7°C) refolding buffer (50 mM Tris-HCI, 10 mM CaCl₂, 50 mM KCI, pH 8.0) until a 1:10 dilution was reached (final volume 10-20 liters). Re-folding occurs over approximately eighteen hours under these conditions. The best results are obtained when the GuHCl extract is slowly added to the refolding buffer over a 2-h period, with gentle mixing. The solution was left undisturbed for at least a 20-h period, and 95% ethanol was added to this solution such that the final ethanol concentration was approximately 20%. This solution was left undisturbed until the flocculated material settled to the bottom, usually not less than sixty minutes. The solution was filtered through a 0.2 um Millipore Millipak 200. This filtration step may be optionally preceded by a centrifugation step. The filtrate was concentrated to 1 to 2 liters using an Amicon spiral cartridge with a 10,000 MWCO cartridge, again at 4°C.

The concentrated crude antigen-binding protein sample was dialyzed against Buffer A (60 mM MOPS, 0.5 mM Ca acetate, pH 6.0-6.4) until the conductivity was lowered to that of Buffer A. The sample was then loaded on a 21.5 x 250-mm polyaspartic acid PolyCAT A column, manufactured by Poly LC of Columbia, Maryland. If more than 60 mg of protein is loaded on this column, the resolution begins to deteriorate; thus, the concentrated crude sample often must be divided into several PolyCAT A runs. Most antigen-binding proteins have an extinction coefficient of about 2.0 ml mg⁻¹ cm⁻¹ at 280 nm and this can be used to determine protein concentration. The antigen-binding protein sample was eluted from the PolyCAT A column with a 50-min linear gradient from Buffer A to Buffer B (see Table 1). Most of the single-chain proteins elute between 20 and 26 minutes when this gradient is used. This corresponds to an eluting solvent composition cf approximately 70% Buffer A and 30% Buffer B. Most of the bivalent antigen-binding proteins elute later than 45 minutes, which correspond to over 90% Buffer B.

Figure 7 is a chromatogram depicting the separation of single-chain protein from bivalent CC49/212 protein, using the cation-exchange method just described. Peak 1, 27.32 minutes, represents the monomeric single-chain fraction. Peak 2, 55.52 minutes, represents the bivalent protein fraction.

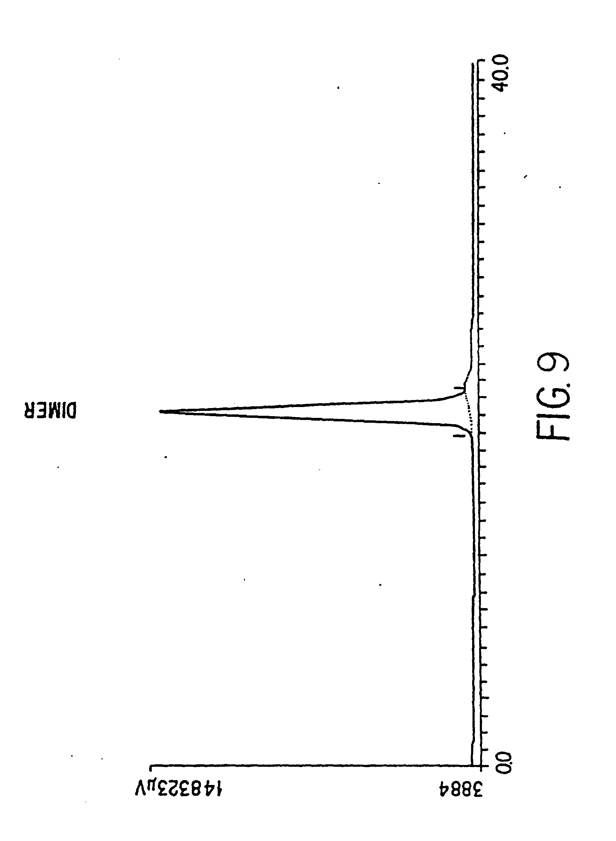

Figure 8 is a chromatogram of the purified monomeric single-chain antigen-binding protein CC49/212 (Fraction 7 from Fig. 7) run on a Waters Protein-Pak 300SW gel filtration column. Monomer, with minor contaminates of dimer and trimer, is shown. Figure 9 is a chromatogram of the purified bivalent antigen-binding protein CC49/212 (Fraction 15 from Fig. 7) run on the same Waters Protein-Pak 300SW gel filtration column as used in Fig. 8.

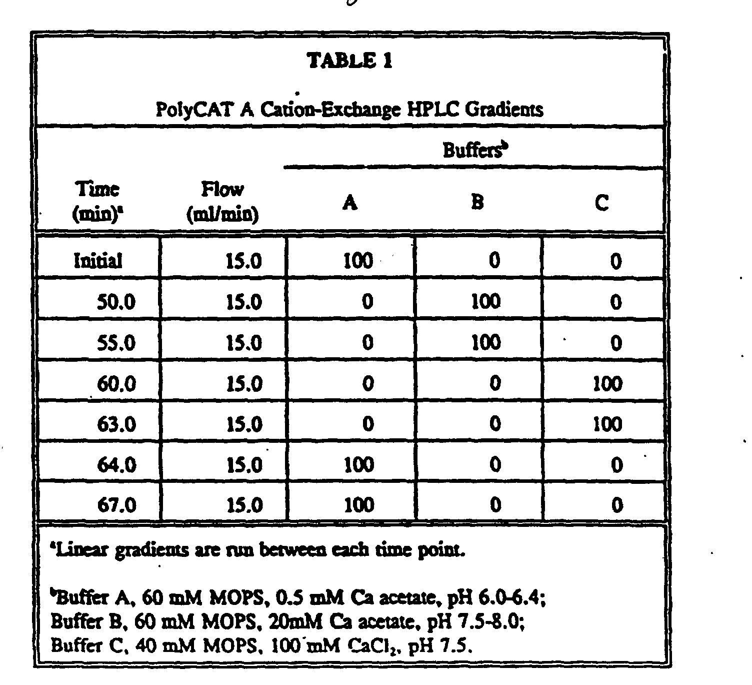

This purification procedure yielded multivalentantigen-binding proteins that are more than 95% pure as examined by SDS-PAGE and size exclusion HPLC. Modifications of the above procedure may be dictated by the isoelectric point of the particular multivalent antigen-binding protein being purified. Of the monomeric single-chain proteins that have been purified to date, all have had an isoelectric point (p1) between 8.0 and 9.5. However, it is possible that a multivalent antigen-binding protein may be produced with a pI of less than 7.0. In that case, an anion exchange column may be required for purification.

The CC49 monoclonal antibody was developed by Dr. Jeffrey Schlom's group, Laboratory of Tumor Immunology and Biology, National Cancer Institute. It binds specifically to the pan-carcinoma tumor antigen TAG-72. See Muraro, R. *et al*., *Cancer Research 48*:4588-4596 (1988).

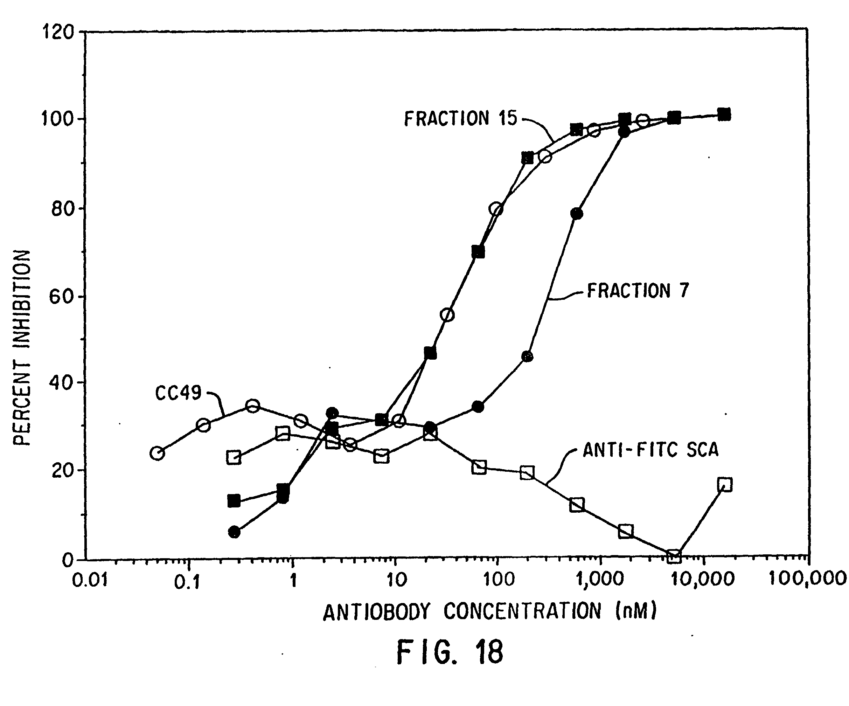

To determine the binding properties of the bivalent and monomeric CC49/212 antigen-binding proteins, a competition radioimmunoassay (RIA) was set up in which a CC49 IgG (with two antigen binding sites) radiolabeled with ¹²⁵I was competed against unlabeled CC49 IgG, or monovalent (fraction 7 in Figure 7) or bivalent (fraction 15 in Figure 7) CC49/212 antigen-binding protein for binding to the TAG-72 antigen on a human breast carcinoma extract. (See Figure 18). This competition RIA showed that the bivalent antigen-binding protein competed equally well for the antigen as did IgG, whereas the monovalent single-chain antigen-binding protein needed a ten-fold higher protein concentration to displace the IgG. Thus, the monovalent antigen-binding protein competes with about a ten-fold lower affinity for the antigen than does the bivalent IgG or bivalent antigen-binding protein. Figure 18 also shows the result of the competition RIA of a non-TAG-72 specific single-chain antigen-binding protein, the antifluorescein 4-4-20/212, which does not compete for binding.

### Example 2

### Process of Making Multivalent

### Antigen-Binding Proteins Using Dissociating Agents

### A. Process Using Guanidine HCl and Ethanol

Multivalent antigen-binding proteins were produced from purified single-chain proteins in the following way. First the purified single-chain protein at a concentration of 0.25-4 mg/ml was dialyzed against 0.5 moles/liter (M) guanidine hydrochloride (GuHCl), 20% ethanol (EtOH), in 0.05 M TRIS, 0.05 M KCI, 0.01 M CaCl₂ buffer pH 8.0. This combination of dissociating agents is thought to disrupt the V_{L}/V_{H} interface, allowing the V_{H} of a first single-chain molecule to come into contact with a V_{L} from a second single-chain molecule. Other dissociating agents such as urea, and alcohols such as isopropanol or methanol should be substitutable for GuHCl and EtOH. Following the initial dialysis, the protein was dialyzed against the load buffer for the final HPLC purification step. Two separate purification protocols, cation exchange and gel filtration chromatography, can be used to separate the single-chain protein monomer from the multivalent antigen-binding proteins. In the first method, monomeric and multivalent antigen-binding proteins were separated by using cation exchange HPLC chromography, using a polyaspartate column (PolyCAT A). This was a similar procedure to that used in the final purification of the antigen-binding proteins as described in Example 1. The load buffer was 0.06 M MOPS, 0.001 M Calcium Acetate pH 6.4. In the second method, the monomeric and multivalent antigen-binding proteins were separated by gel filtration HPLC chromatography using as a load buffer 0.04 M MOPS, 0.10 M Calcium Acetate pH 7.5. Gel filtration chromatography separates proteins based on their molecular size.

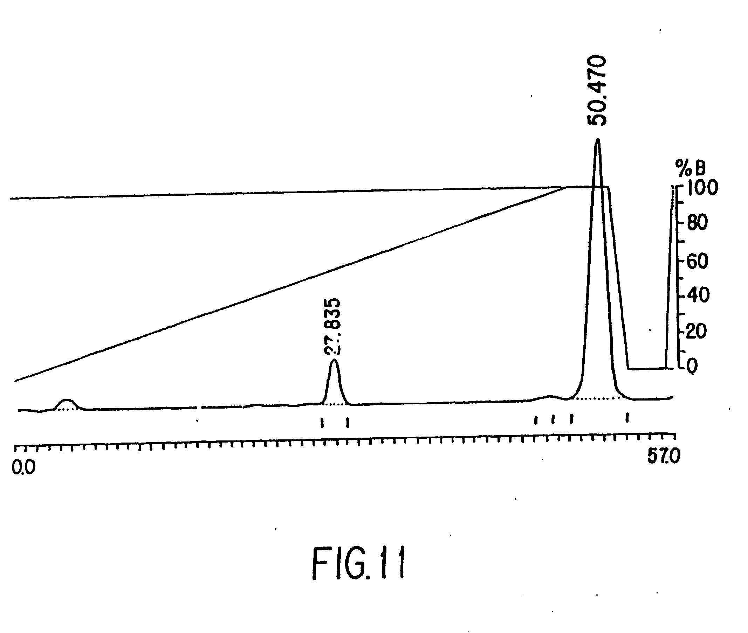

Once the antigen-binding protein sample was loaded on the cation exchange HPLC column, a linear gradient was run between the load buffer (0.04 to 0.06 M MOPS, 0.000 to 0.001 M calcium acetate, 0 to 10% glycerol pH 6.0-6.4) and a second buffer (0.04 to 0.06 M MOPS, 0.01 to 0.02 M calcium acetate, 0 to 10% glycerol pH 7.5). It was important to have extensively dialyze the antigen-binding protein sample before loading it on the column. Normally, the conductivity of the sample is monitored against the dialysis buffer. Dialysis is continued until the conductivity drops below 600 µS. Figure 11 shows the separation of the monomeric (27.83 min.) and bivalent (50.47 min.) forms of the CC49/212 antigen-binding protein by cation exchange. The chromatographic conditions for this separation were as follows: PolyCAT A column, 200 x 4.6mm, operated at 0.62 ml/min.; load buffer and second buffer as in Example 1; gradient program from 100 percent load buffer A to 0 percent load buffer A over 48 mins; sample was CC49/212, 1.66 mg/ml; injection volume 0.2 ml. Fractions were collected from the two peaks from a similar chromatogram and identified as monomeric and bivalent proteins using gel filtration HPLC chromatography as described below.

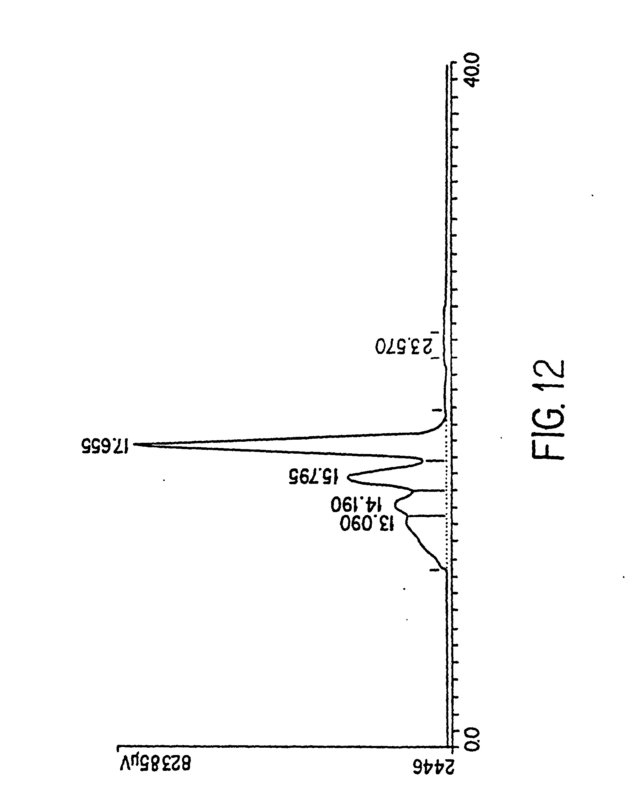

Gel filtration HPLC chromatography (TSK G2000SW column from Toyo Soda, Tokyo, Japan) was used to identify and separate monomeric single-chain and multivalent antigen-binding proteins. This procedure has been described by Fukano, *et al*., *J. Chromatography* **166:**47 (1978). Multimerization (creation of multivalent protein from monomeric single-chain protein) was by treatment with 0.5 M GuHCl and 20% EtOH for the times indicated in Table 2A followed by dialysis into the chromatography buffer. Figure 12 shows the separation of monomeric (17.65 min.), bivalent (15.79 min.), trivalent (14.19 min.), and higher oligomers (shoulder at about 13.09 min.) of the B6.2/212 antigen-binding protein. The B6.2/212 single-chain antigen-binding protein is described in Colcher, D., *et al*., *J*. *Nat. Cancer Inst. 82*:1191-1197 (1990)). This separation depicts the results of a 24-hour multimerization treatment of a 1.0 mg/ml B6.2/212 antigen-binding protein sample. The HPLC buffer used was 0.04 M MOPS, 0.10 M calcium acetate, 0.04% sodium azide, pH 7.5.

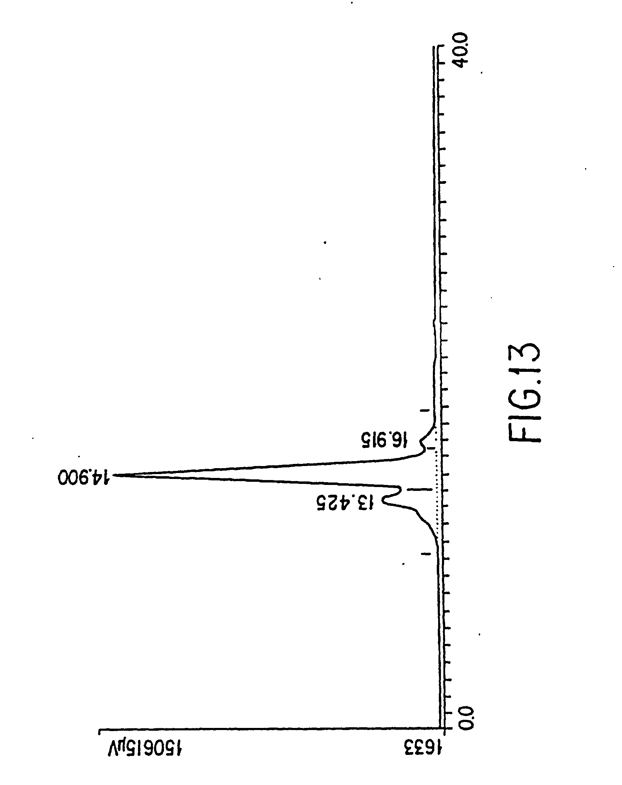

Figure 13 shows the results of a 24-hour treatment of a 4.0 mg/ml CC491212 antigen-binding protein sample, generating monomeric, bivalent and trivalent proteins at 16.91, 14.9, and 13.42 min., respectively. The HPLC buffer was 40 mM MOPS, 100 mM calcium acetate, pH 7.35. Multimerization treatment was for the times indicated in Table 2.

The results of Example 2A are shown in Table 2A. Table 2A shows the percentage of bivalent and other multivalent forms before and after treatment with 20% ethanol and 0.5M GuHCl. Unless otherwise indicated, percentages were determined using a automatic data integration software package.

**Table 2A**

| Summary of the generation of bivalent and higher multivalent forms of B6.2/212 and CC49/212 proteins using guanidine hydrochloride and ethanol | | | | | | |
|---|---|---|---|---|---|---|
| | Time | Concentration | % | | | |
| protein | (hours) | (mg/ml) | monomer | dimer | trimer | multimers |
| CC49/212 | 0 | 0.25 | 86.7 | 11.6 | 1.7 | 0.0 |
| | 0 | 1.0² | 84.0 | 10.6 | 5.5 | 0.0 |
| | 0 | 4.0 | 70.0 | 17.1 | 12.9¹ | 0.0 |
| | 2 | 0.25² | 62.9 | 33.2 | 4.2 | 0.0 |
| | 2 | 1.0 | 24.2 | 70.6 | 5.1 | 0.0 |
| | 2 | 4.0 | 9.3 | 81.3 | 9.5 | 0.0 |
| | 26 | 0.25 | 16.0 | 77.6 | 6.4 | 0.0 |
| | 26 | 1.0 | 9.2 | 82.8 | 7.9 | 0.0 |
| | 26 | 4.0 | 3.7 | 78.2 | 18.1 | 0.0 |
| B6.2/212 | 0 | 0,25 | 100.0 | 0.0 | 0.0 | 0.0 |
| | 0 | 1,0 | 100.0 | 0.0 | 0.0 | 0.0 |
| | 0 | 4.0 | 100.0 | 0.0 | 0.0 | 0.0 |
| | 2 | 0.25² | 98.1 | 1.9 | 0.0 | 0.0 |
| | 2 | 1.0 | 100.0 | 0.0 | 0.0 | 0.0 |
| | 2 | 4.0 | 90.0 | 5.5 | 1.0 | 0.0 |
| | 24 | 0.25 | 45.6 | 37.5 | 10.2 | 6.7 |
| | 24 | 1.0 | 50.8 | 21.4 | 12.3 | 15.0 |
| | 24 | 4.0 | 5.9 | 37.2 | 25.7 | 29.9 |

| | | | | | | |
|---|---|---|---|---|---|---|
| ¹ Based on cut out peaks that were weighted. | | | | | | |
| ² Average of two experiments. | | | | | | |

### B. Process Using Urea and Ethanol

Multivalent antigen-binding proteins were produced from purified single-chain proteins in the following way. First the purified single-chain protein at a concentration of 0.25-1 mg/ml was dialyzed against 2M urea, 20% ethanol (EtOH), and 50mM Tris buffer pH 8.0, for the times indicated in Table 2B. This combination of dissociating agents is thought to disrupt the V_{L}/V_{H} interface, allowing the V_{H} of a first single-chain molecule to come into contact with a V_{L} from a second single-chain molecule. Other dissociating agents such as isopropanol or methanol should be substitutable for EtOH. Following the initial dialysis, the protein was dialyzed against the load buffer for the final HPLC purification step.

Gel filtration HPLC chromatography (TSK G2000SW column from Toyo Soda, Tokyo, Japan) was used to identify and separate monomeric single-chain and multivalent antigen-binding proteins. This procedure has been described by Fukano, *et al*., *J*. *Chromatography* **166:**47 (1978).

The results of Example 2B are shown in Table 2B. Table 2B shows the percentage of bivalent and other multivalent forms before and after treatment with 20% ethanol and urea. Percentages were determined using an automatic data integration software package.

**Table 2B**

| Summary of the generation of bivalent and higher multivalent forms of B6.2/212 and CC49/212 proteins using urea and ethanol | | | | | | |
|---|---|---|---|---|---|---|
| protein | Time | Concentration | % | | | |
| | (hours) | (mg/ml) | monomer | dimer | trimer | multimers |
| B6.2 | 0 | 0.25 | 44.1 | 37.6 | 15.9 | 2.4 |
| | 0 | 1.0 | 37.7 | 33.7 | 19.4 | 9.4 |
| | 3 | 0.25 | 22.2 | 66.5 | 11,3 | 0.0 |
| | 3 | 1.0 | 13.7 | 69.9 | 16.4 | 0.0 |

### Example 3

### Determination of Binding Constants

Three anti-fluorescein single-chain antigen-binding proteins have been constructed based on the anti-fluorescein monoclonal antibody 4-4-20. The three 4-4-20 single-chain antigen-binding proteins differ in the polypeptide linker connecting the V_{H} and V_{L} regions of the protein. The three linkers used were 202', 212 and 216 (see Table 3). Bivalent and higher forms of the 4-4-20 antigen-binding protein were produced by concentrating the purified monomeric single-chain antigen-binding protein in the cation exchange load buffer (0.06 M MOPS, 0.001 M calcium acetate pH 6.4) to 5 mg/ml. The bivalent and monomeric forms of the 1-4-20 antigen-binding proteins were separated by cation exchange HPLC (polyaspartate column) using a 50 min. linear gradient between the load buffer (0.06 M MOPS, 0.001 M calcium acetate pH 6.4) and a second buffer (0.06 M MOPS, 0.02 M calcium acetate pH 7.5). Two 0.02 ml samples were separated, and fractions of the bivalent and monomeric protein peaks were collected on each run. The amount of protein contained in each fraction was determined from the absorbance at 278 nm from the first separation. Before collecting the fractions from the second separation run, each fraction tube had a sufficient quantity of 1.03 x 10⁵ M fluorescein added to it, such that after the fractions were collected a 1-to-1 molar ratio of protein-to-fluorescein existed. Addition of fluorescein stabilized the bivalent form of the 4-4-20 antigen-binding proteins. These samples were kept at 2°C (on ice).

The fluorescein dissociation rates were determined for each of these samples following the procedures described by Herron, J.N., in *Fluorescence Hapten: An Immunological Probe,* E.W. Voss, Ed., CRC Press, Boca Raton, FL (1984). A sample was first diluted with 20 mM HEPES buffer pH 8.0 to 5.0 x 10⁻⁸ M 4-4-20 antigen-binding protein. 560 µl of the 5.0 x 10⁻⁸ M 4-4-20 antigen-binding protein sample was added to a cuvette in a fluorescence spectrophotometer equilibrated at 2°C and the fluorescence was read. 140 µl of 1.02 X 10⁻⁵ M fluoresceinamine was added to the cuvette, and the fluorescence was read every 1 minute for up to 25 minutes (see Table 4).

The binding constants (Kₐ) for the 4-4-20 single-chain antigen-binding protein monomers diluted in 20 mM HEPES buffer pH 8.0 in the absence of fluorescein were also determined (see Table 4).

The three polypeptide linkers in these experiments differ in length. The 202', 212 and 216 linkers are 12, 14 and 18 residues long, respectively. These experiments show that there are two effects of linker length on the 4-4-20 antigen-binding proteins: first, the shorter the linker length the higher the fraction of bivalent protein formed; second, the fluorescein dissociation rates of the monomeric single-chain antigen-binding proteins are effected more by the linker length than are the dissociation rates of the bivalent antigen-binding proteins. With the shorter linkers 202' and 212, the bivalent antigen-binding proteins have slower dissociation rates than the monomers. Thus, the linkers providing optimum production and binding affinities for monomeric and bivalent antigen-binding proteins may be different. Longer linkers may be more suitable for monomeric single-chain antigen-binding proteins, and shorter linkers may be more suitable for multivalent antigen-binding proteins.

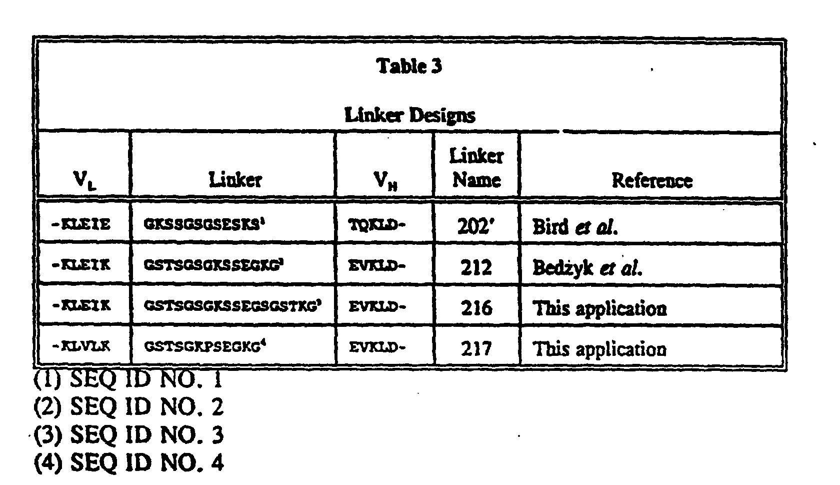

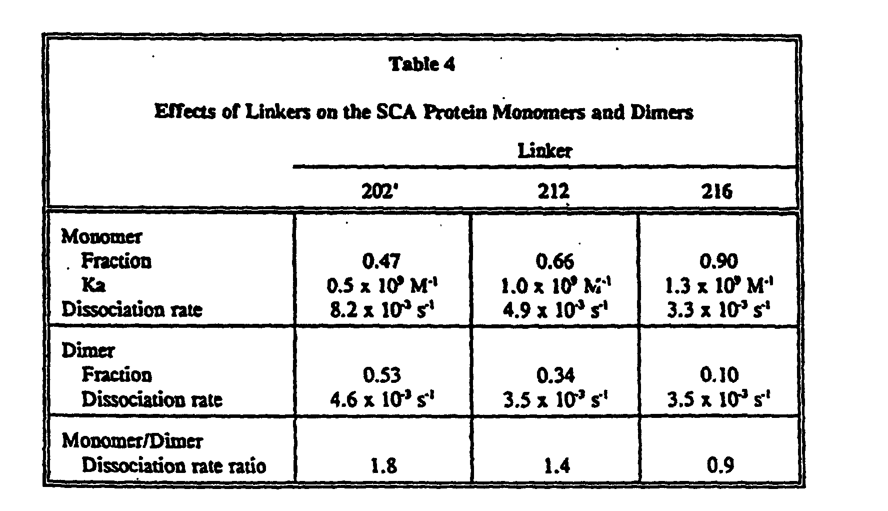

### Example 4

### Genetic Construction of a Mixed-Fragment Bivalent Antigen-Binding Protein

The genetic constructions for one particular heterobivalent antigen-binding protein according to the Rearrangement model are shown in Figures 10A and 10B. Figure 10A is an amino acid and nucleotide sequence listing of the 4-4-20 V_{L}/212/CC49 V_{H} construct, coding for a single-chain protein with a 4-4-20 V_{L}, linked via a 212 polypeptide linker to a CC49 V_{H}. Figure 10B is a similar listing showing the CC49 V_{L}/212/4-4-20 V_{H} construct, coding for a single-chain protein with a CC49 V_{L}, linked via a 212 linker to a 4-4-20 V_{H}. These single-chain proteins may recombine according to the Rearrangement model to generate a heterobivalent protein comprising a CC49 antigen-binding site linked to a 4-4-20 antigen-binding site, as shown in Figure 5B.

"4-4-20 V_{L}" means the variable region of the light chain of the 4-4-20 mouse monoclonal antibody (Bird, R.E. *et al*., *Science* **242:**423 (1988)). The number "212" refers to a specific 14-residue polypeptide linker that links the 4-4-20 V_{L} and the CC49 V_{H}. See *Bedzyk*, *W.D.* et al., *J. Biol. Chem. 265:18615-18620 (1990).* "CC49 V_{H}" is the variable region of the heavy chain of the CC49 antibody, which binds to the TAG-72 antigen. The CC49 antibody was developed at The National Institutes of Health by Schlom, et al. *Generation and Characterization of B72.3 Second Generation Monoclonal Antibodies Reactive With The Tumor-associated Glycoprotein 72 Antigen*, Cancer Research **48:**4588-4596 (1988).

Insertion of the sequences shown in FIGS. 10A and 10B, by standard recombinant DNA methodology, into a suitable plasmid vector will enable one of ordinary skill in the art to transform a suitable host for subsequent expression of the single-chain proteins. See Maniatis et al., *Molecular Cloning, A Laboratory Manual*, p. 104, Cold Spring Harbor Laboratory (1982), for general recombinant techniques for accomplishing the aforesaid goals; see also U.S. Patent 4,946,778 (Ladner *et al*.) for a complete description of methods of producing single-chain protein molecules by recombinant DNA technology.

To produce multivalent antigen-binding proteins from the two single-chain proteins, 4-4-20V_{L}-212/CC49V_{H} and CC49V_{L}/212/4-4-20V_{H}, the two single-chain proteins are dialyzed into 0.5 M GuHCl/20% EtOH being combined in a single solution either before or after dialysis. The multivalent proteins are then produced and separated as described in Example 2.

### Example 5

### Preparation of Multivalent Antigen-Binding Proteins by Chemical Cross-Linking

Free cysteines were engineered into the C-terminal of the 4-4-20/212 single-chain antigen-binding protein, in order to chemically crosslink the protein. The design was based on the hinge region found in antibodies between the C_{H}1 and C_{H}2 regions. In order to try to reduce antigenicity in humans, the hinge sequence of the most common IgG class, IgG1, was chosen. The 4-4-20 Fab structure was examined and it was determined that the C-terminal sequence GluH216-ProH217-ArgH218, was part of the C_{H}1 region and that the hinge between C_{H}1 and C_{H}2 starts with ArgH218 or GlyH219 in the mouse 4-4-20 IgG2A antibody. Figure 14 shows the structure of a human IgG. The hinge region is indicated generally. Thus the hinge from human IgG1 would start with LysH218 or SerH219. (See Table 5).

The C-terminal residue in most of the single-chain antigen-binding proteins described to date is the amino acid serine. In the design for the hinge region, the C-terminal serine in the 4-4-20/212 single-chain antigen-binding protein was made the first serine of the hinge and the second residue of the hinge was changed from a cysteine to a serine. This hinge cysteine normally forms a disulfide bridge to the C-terminal cysteine in the light chain.

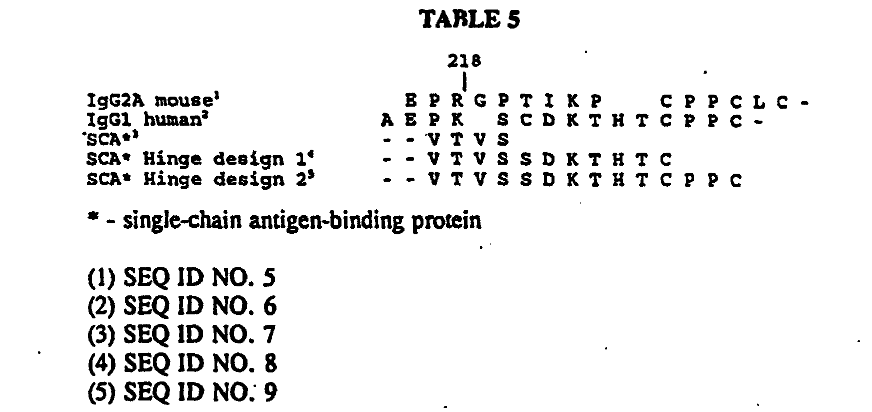

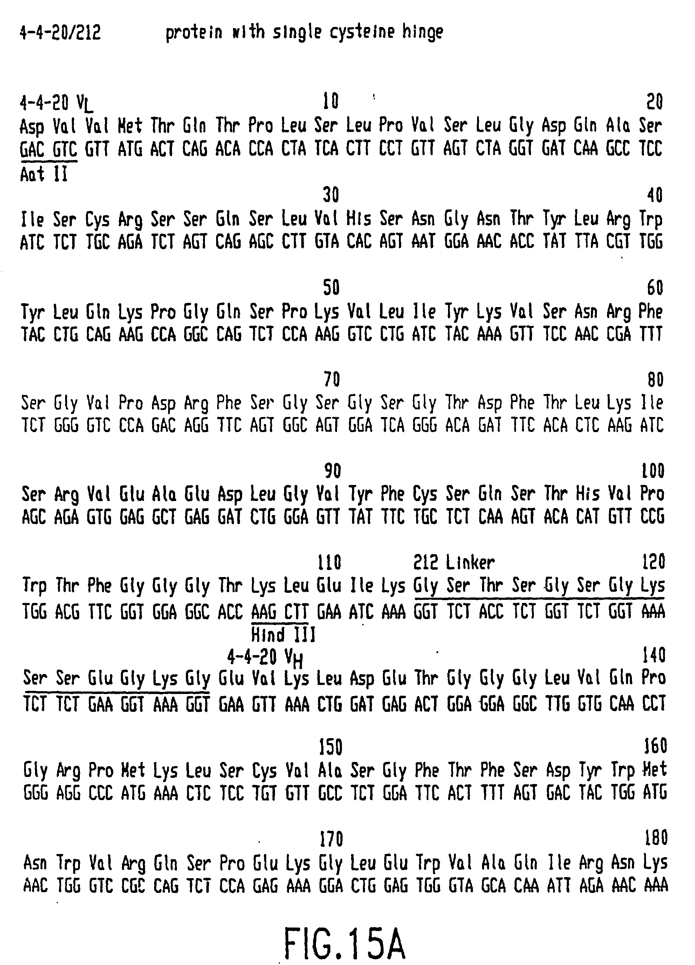

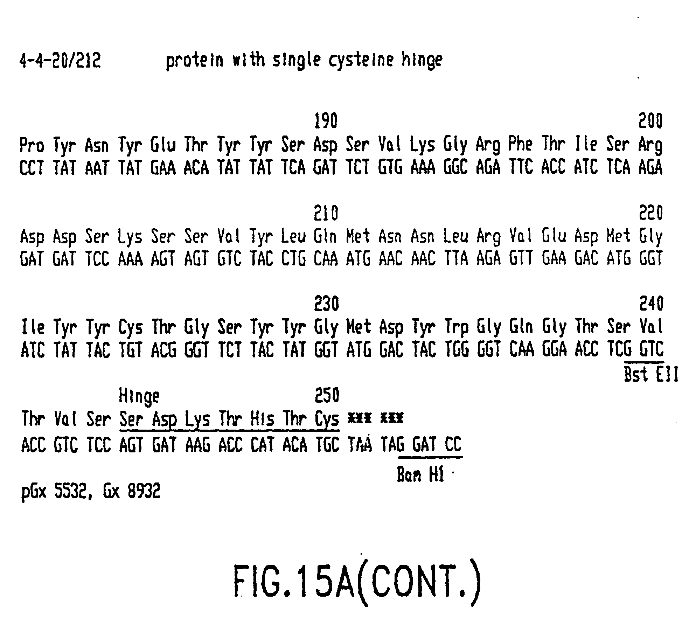

There are possible advantages to having two C-terminal cysteines, for they might form an intramolecular disulfide bond, making the protein recovery easier by protecting the sulfurs from oxidation. The hinge regions were added by introduction of a BstE II restriction site in the 3'-terminus of the gene encoding the 4-4-20/212 single-chain antigen-binding protein (see Figures 15A-15B).

The monomeric single-chain antigen-binding protein containing the C-terminal cysteine can be purified using the normal methods of purifying a single-chain antigen-binding proteins, with minor modifications to protect the free sulfhydryls. The cross-linking could be accomplished in one of two ways. First, the purified single-chain antigen-binding protein could be treated with a mild reducing agent, such as dithiothreitol, then allowed to air oxidize to form a disulfide-bond between the individual single-chain antigen-binding proteins. This type of chemistry has been successful in producing heterodimers from whole antibodies (Nisonoff *et al*., Quantitative Estimation of the Hybridization of Rabbit Antibodies, *Nature 4826*:355-359 (1962); Brennan *et al*., Preparation of Bispecific Antibodies by Chemical Recombination of Monoclonal Immunoglobulin G₁ Fragments, *Science 229*:81-83 (1985)). Second, chemical crosslinking agents such as bismaleimidehexane could be used to cross-link two single-chain antigen-binding proteins by their C-terminal cysteines. See Partis *et al*., *J. Prot. Chem. 2*:263-277 (1983).

### Example 6

### Genetic Construction of Bivalent Antigen-Binding Proteins

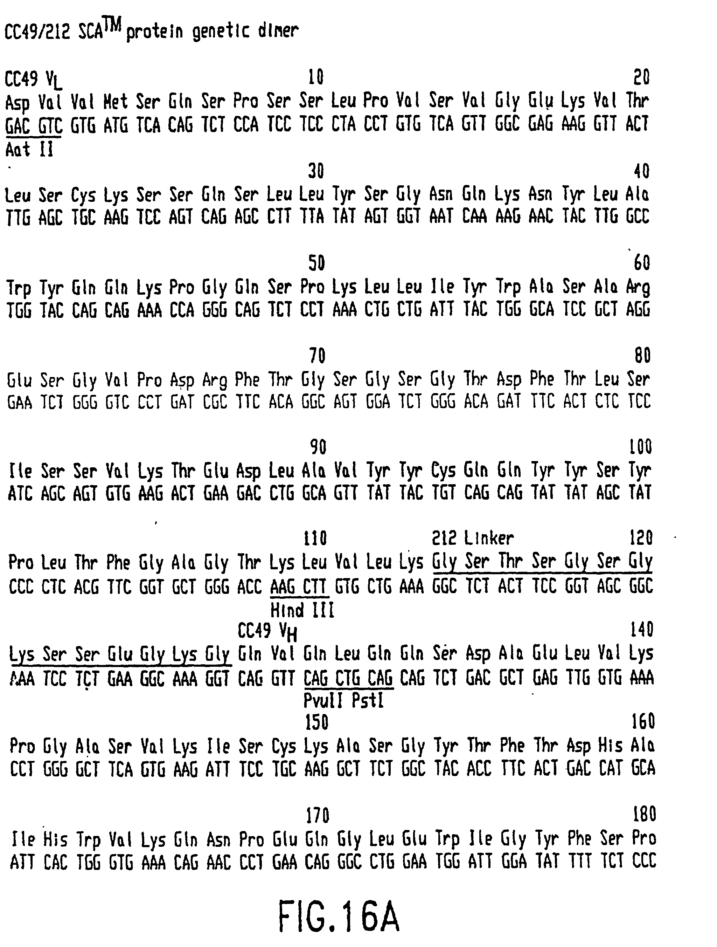

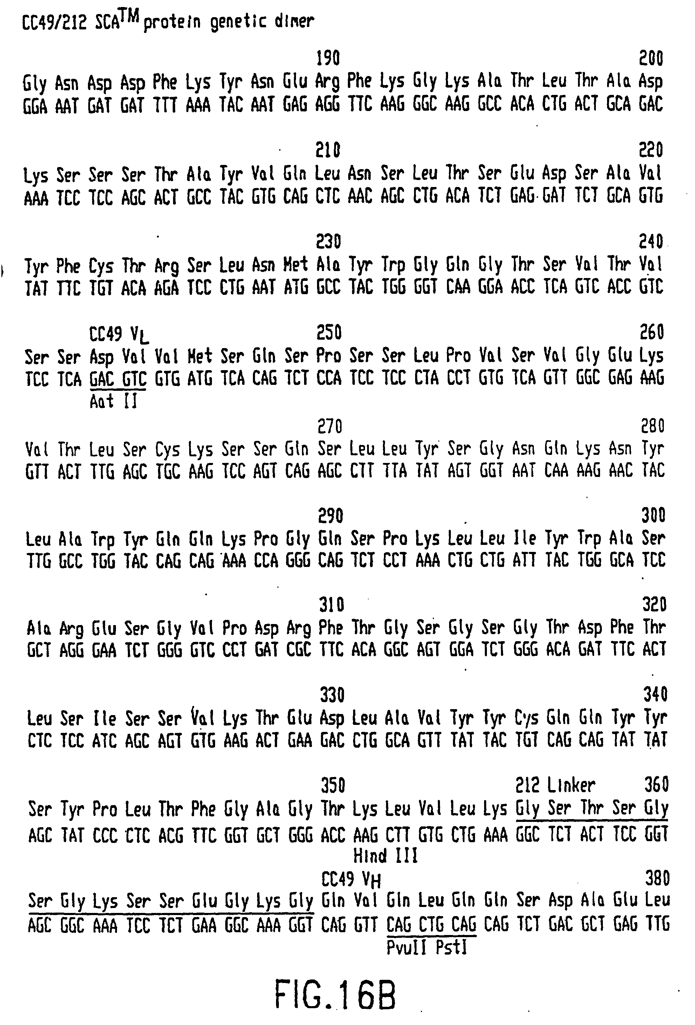

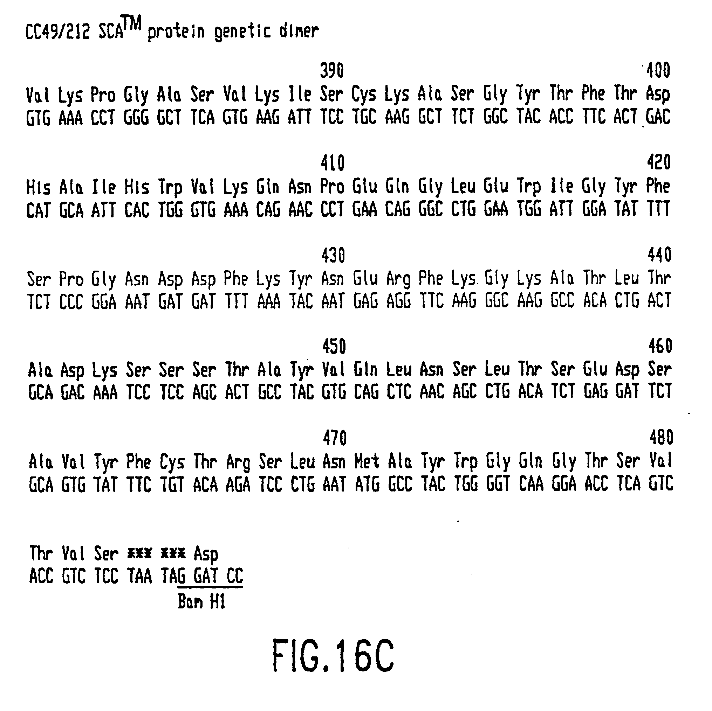

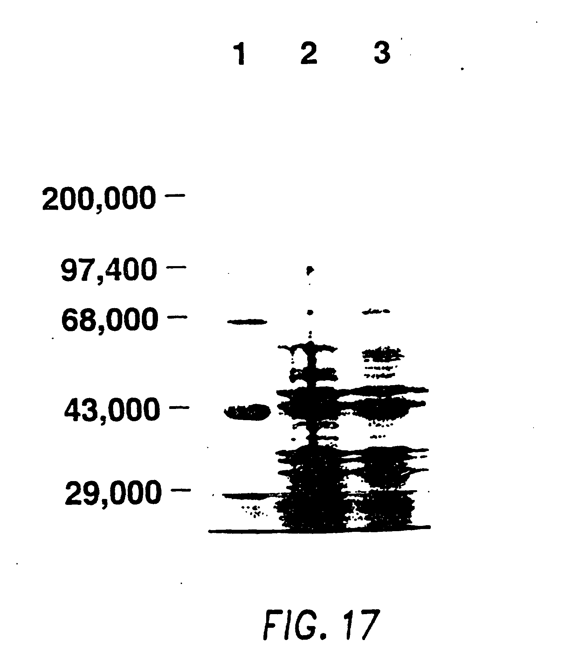

Bivalent antigen-binding proteins can be constructed genetically and subsequently expressed in *E. coli* or other known expression systems. This can be accomplished by genetically removing the stop codons at the end of a gene encoding a monomeric single-chain antigen-binding protein and inserting a linker and a gene encoding a second single-chain antigen-binding protein. We have constructed a gene for a bivalent CC49/212 antigen-binding protein in this manner (see Figure 16). The CC49/212 gene in the starting expression plasmid is in an Aat II to Bam HI restriction fragment (see Bird *et al*., Single-Chain Antigen-Binding Proteins, *Science 242*:423-426 (1988); and Whitlow *et al*., Single-Chain Fᵥ Proteins and Their Fusion Proteins, *Methods* **2**:97-105 (1991)). The two stop codons and the Bam HI site at the C-terminal end of the CC49/212 antigen-binding protein gene were replaced by a single residue linker (Ser) and an Aat II restriction site. The resulting plasmid was cut with Aat II and the purified Aat II to Aat II restriction fragment was ligated into Aat II cut CC49/212 single-chain antigen-binding protein expression plasmid. The resulting bivalent CC49/212 single-chain antigen-binding protein expression plasmid was transfected into an *E. coli* expression host that contained the gene for the cI857 temperature-sensitive repressor. Expression of single-chain antigen-binding protein in this system is induced by raising the temperature from 30°C to 42°C. Fig. 17 shows the expression of the divalent CC49/212 single-chain antigen-binding protein of Fig. 16 at 42°C, on an SDS-PAGE gel containing total *E. coli* protein. Lane 1 contains the molecular weight standards. Lane 2 is the uninduced *E. coli* production strain grown at 30°C. Lane 3 is divalent CC49/212 single-chain antigen-binding protein induced by growth at 42°C. The arrow shows the band of expressed divalent CC49/212 single-chain antigen-binding protein.

### Example 7

### Construction, Purification, and Testing of 4-4-20/CC49 Heterodimer F_{V} With 217 Linkers.

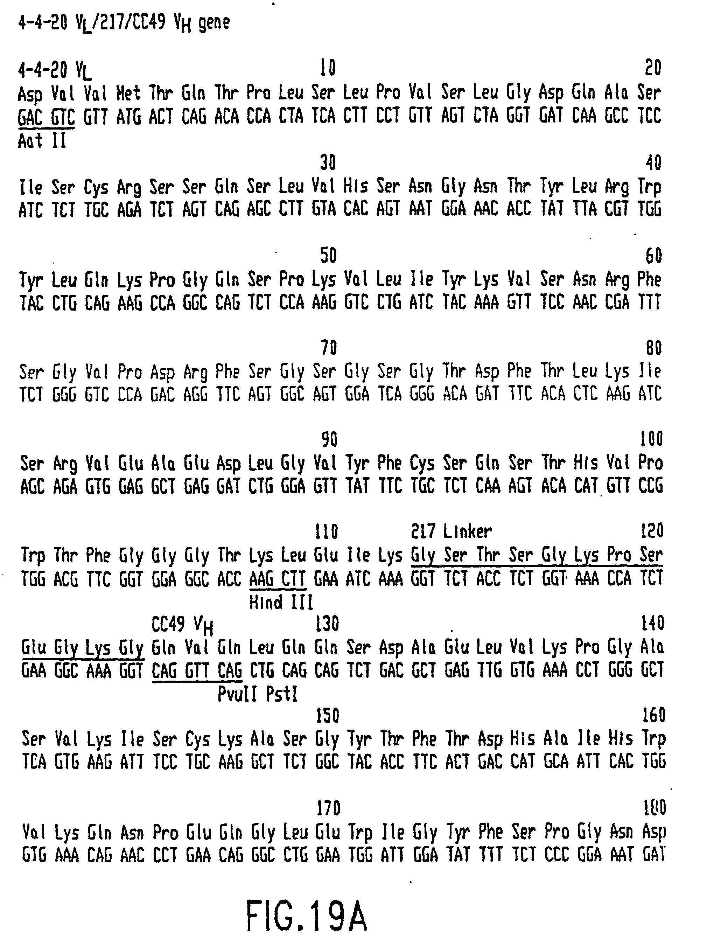

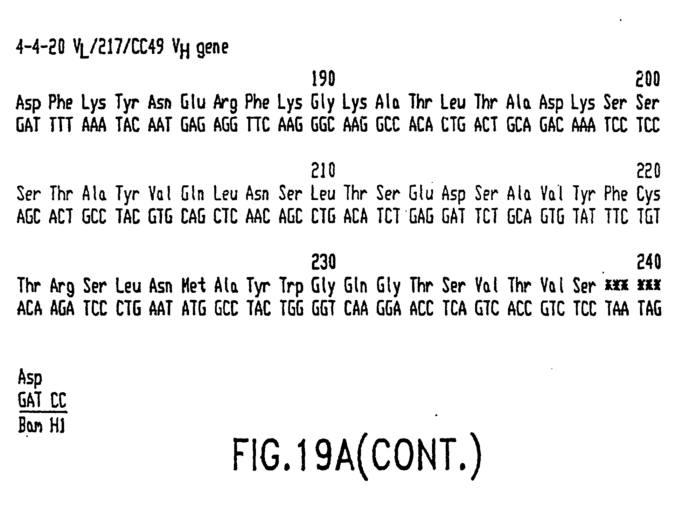

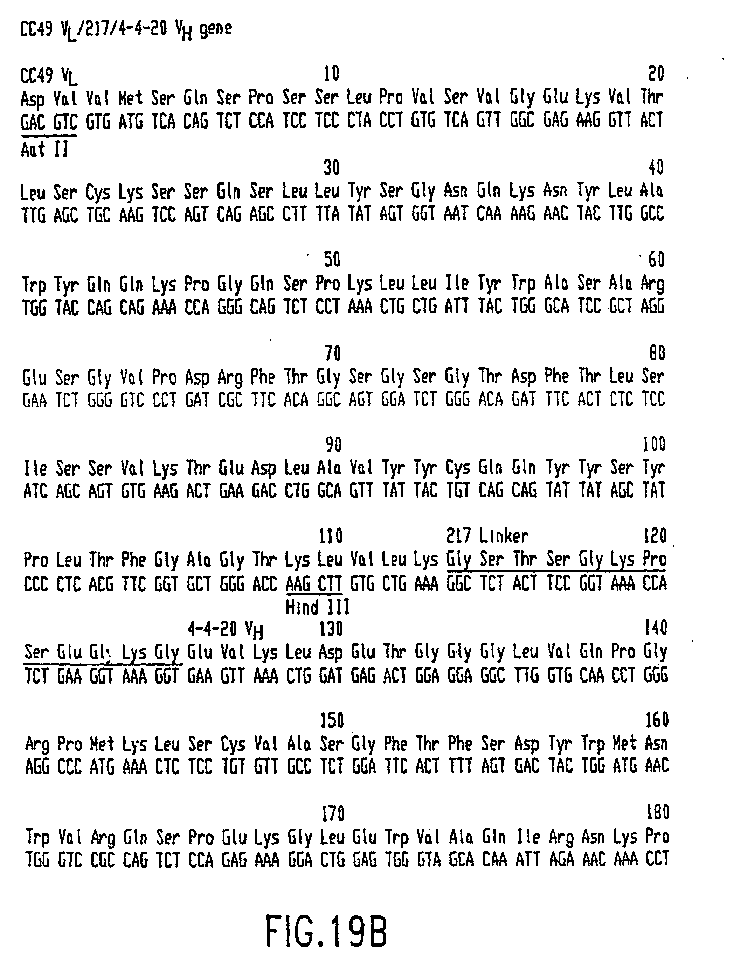

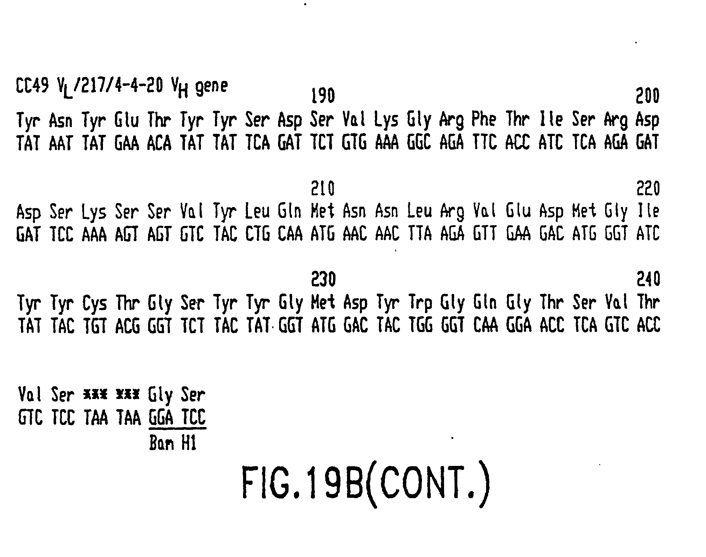

The goals of this experiment were to produce, purify and analyze for activity a new heterodimer Fv that would bind to both fluorescein and the pan-carcinoma antigen TAG-72. The design consisted of two polypeptide chains, which associated to form the active heterodimer Fv. Each polypeptide chain can be described as a mixed single-chain Fv (mixed sFv). The first mixed sFv (GX 8952) comprised a 4-4-20 variable light chain (V_{L}) and a CC-49 variable heavy chain (V_{H}) connected by a 217 polypeptide linker (Figure 19A). The second mixed sFv (GX 8953) comprised a CC-49 V_{L} and a 4-4-20 V_{H} connected by a 217 polypeptide linker (Figure 19B). The sequence of the 217 polypeptide linker is shown in Table 3. Construction of analogous CC49/4-4-20 heterodimers connected by a 212 polypeptide linker as described in Example 4.

### Results

### A. Purification

One 10-liter fermentation of each mixed sFv was grown on casein digest-glucose-salts medium at 32°C to an optical density at 600 nm of 15 to 20. The mixed sFv expression was induced by raising the temperature of the fermentation to 42°C for one hour. 277gm (wet cell weight) of *E. coli* strain GX 8952 and 233gm (wet cell weight) of *E. coli* strain GX 8953 were harvested in a centrifuge at 7000g for 10 minutes. The cell pellets were kept and the supernate discarded. The cell pellets were frozen at -20°0C for storage.

2.55 liters of "lysis/wash buffer" (50mM Tris/ 200mM NaCl/ 1 mM EDTA, pH 8.0) was added to both of the mixed sFv's cell pellets, which were previously thawed and combined to give 510gm of total wet cell weight. After complete suspension of the cells they were then passed through a Gaulin homogenizer at 9000psi and 4°C. After this first pass the temperature increased to 23°C. The temperature was immediately brought down to 0°C using dry ice and methanol. The cell suspension was passed through the Gaulin homogenizer a second time and centrifuged at 8000 rpm with a Dupont GS-3 rotor for 60 minutes. The supernatant was discarded after centrifugation and the pellets resuspended in 2.5 liters of "lysis/wash buffer" at 4°C. This suspension was centrifuged for 45 minutes at 8000 rpm with the Dupont GS-3 rotor. The supernatant was again discarded and the pellet weighed. The pellet weight was 136.1 gm.

1300ml of 6M Guanidine Hydrochloride/50mM Tris/50mM KCl/10mM CaCl₂pH 8.0 at 4°C was added to the washed pellet. An overhead mixer was used to speed solubilization. After one hour of mixing, the heterodimer GuHCl extract was centrifuged for 45 minutes at 8000 rpm and the pellet was discarded. The 1425ml of heterodimer Fv 6M GuHCl extract was slowly added (16 ml/min) to 14.1 liters of "Refold Buffer" (50mM Tris/50mM KCl/10mM CaCl₂, pH 8.0) under constant mixing at 4°C to give an approximate dilution of 1:10. Refolding took place overnight at 4°C.

After 17 hours of refolding the anti-fluorescein activity was checked by a 40% quenching assay, and the amount of active protein calculated. 150mg total active heterodimer Fv was found by the 40% quench assay, assuming a 54,000 molecular weight.

4 liters of prechilled (4°C) 190 proof ethanol was added to the 15 liters of refolded heterodimer with mixing for 3 hours. The mixture sat overnight at 4°C. A flocculent precipitate had settled to the bottom after this overnight treatment. The nearly clear solution was filtered through a Millipak-200 (0.22µ) filter so as to not disturb the precipitate. A 40% quench assay showed that 10% of the anti-fluorescein activity was recovered in the filtrate.

The filtered sample of heterodimer was dialyzed, using a Pellicon system containing 10,000 dalton MWCO membranes, with "dialysis buffer" 40mM MOPS/0.5mM Calcium Acetate (CaAc), pH 6.4 at 4°C. 20 liters of dialysis buffer was required before the conductivity of the retentate was equal to that of the dialysis buffer (~500µS). After dialysis the heterodimer sample was filtered through a Millipak-20 filter, 0.22µ. After this step a 40% quench assay showed there was 8.8 mg of active protein.

The crude heterodimer sample was loaded on a Poly CAT A cation exchange column at 20ml/min. The column was previously equilibrated with 60mM MOPS, 1 mM CaAc pH 6.4, at 4°C, (Buffer A). After loading, the column was washed with 150ml of "Buffer A" at 15ml/min. A 50min linear gradient was performed at 15ml/min using "Buffer A" and "Buffer B" (60mM MOPS, 20mM CaAc pH 7.5 at 4°C). The gradient conditions are presented in Table 6. "Buffer C" comprises 60mM MOPS, 100mM CaCl₂, pH 7.5.

**Table 6**

| Time | %A | %B | %C | Flow |
|---|---|---|---|---|
| 0:00 | 100.0 | 0.0 | 0.0 | 15ml/min |
| 50:00 | 0.0 | 100.0 | 0.0 | 15ml/min |
| 52:00 | 0.0 | 100.0 | 0.0 | 15ml/min |
| 54:00 | 0.0 | 0.0 | 100.0 | 15ml/min |
| 58:00 | 0.0 | 0.0 | 100.0 | 15ml/min |
| 60:00 | 100.0 | 0.0 | 0.0 | 15ml/min |

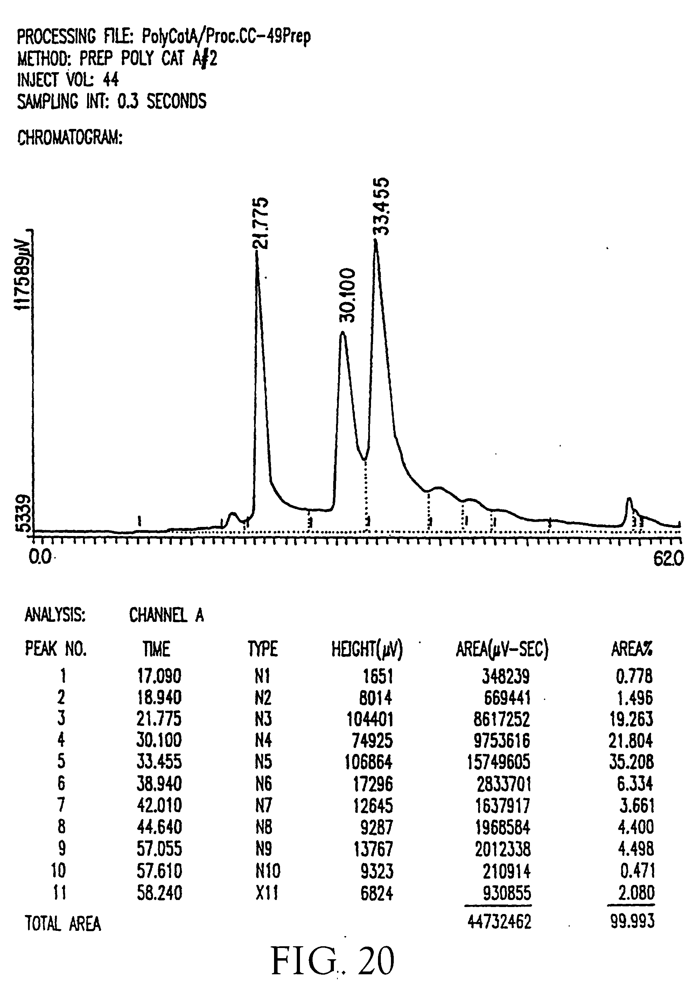

Approximately 50ml fractions were collected and analyzed for activity, purity, and molecular weight by size-exclusion chromatography. The fractions were not collected by peaks, so contamination between peaks is likely. Fractions 3 through 7 were pooled (total volume - 218ml), concentrated to 50ml and dialyzed against 4 liters of 60mM MOPS, 0.5mM CaAc pH 6.4 at 4°C overnight. The dialyzed pool was filtered through a 0.22µ filter and checked for absorbance at 280nm. The filtrate was loaded onto the PolyCAT A column, equilibrated with 60mM MOPS, 1 mM CaAc pH 6.4 at 4°C, at a flow rate of 10ml/min. Buffer B was changed to 60mM MOPS, 10mM CaAc pH 7.5 at 4°C. The gradient was run as in Table 6. The fractions were collected by peak and analyzed for activity, purity, and molecular weight. The chromatogram is shown in Figure 20. Fraction identification and analysis is presented in Table 7.

**Table 7**

| Fraction Analysis of the Heterodimer Fv protein | | | |
|---|---|---|---|
| Fraction No. | A₂₈₀ reading | Total Volume (ml) | HPLC-SE Elution Time (min) |
| 2 | 0.161 | 36 | 20.525 |
| 3 | 0.067 | 40 | |
| 4 | 0.033 | 40 | |
| 5 | 0.178 | 45 | 19.133 |
| 6 | 0.234 | 50 | 19.163 |
| 7 | 0.069 | 50 | |
| 8 | 0.055 | 40 | |

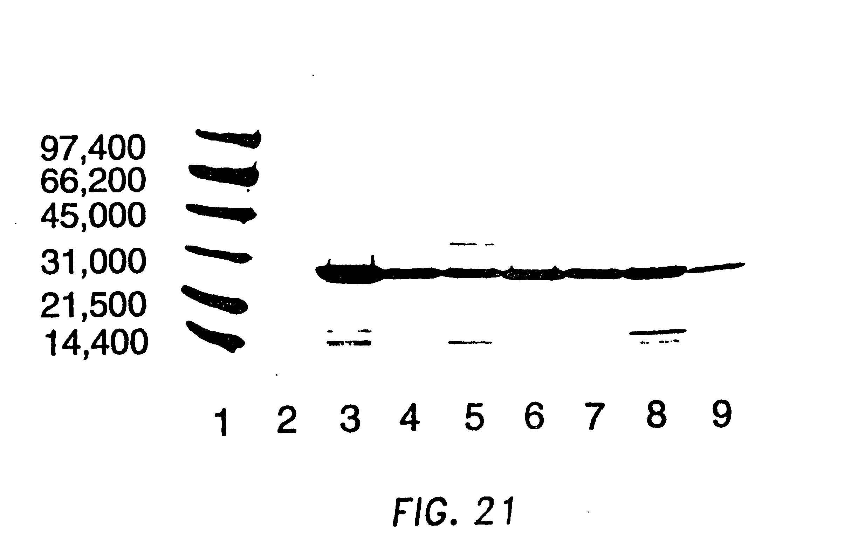

Fractions 2 to 7 and the starting material were analyzed by SDS gel electrophoresis, 4-20%. A picture and description of the gel is presented in Figure 21.

### B. HPLC Size Exclusion Results

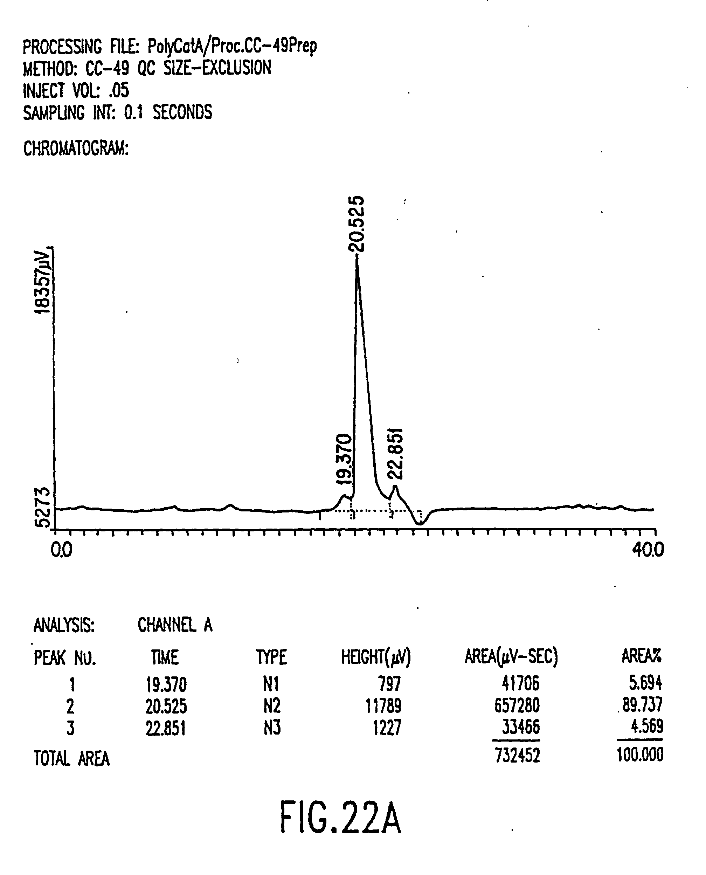

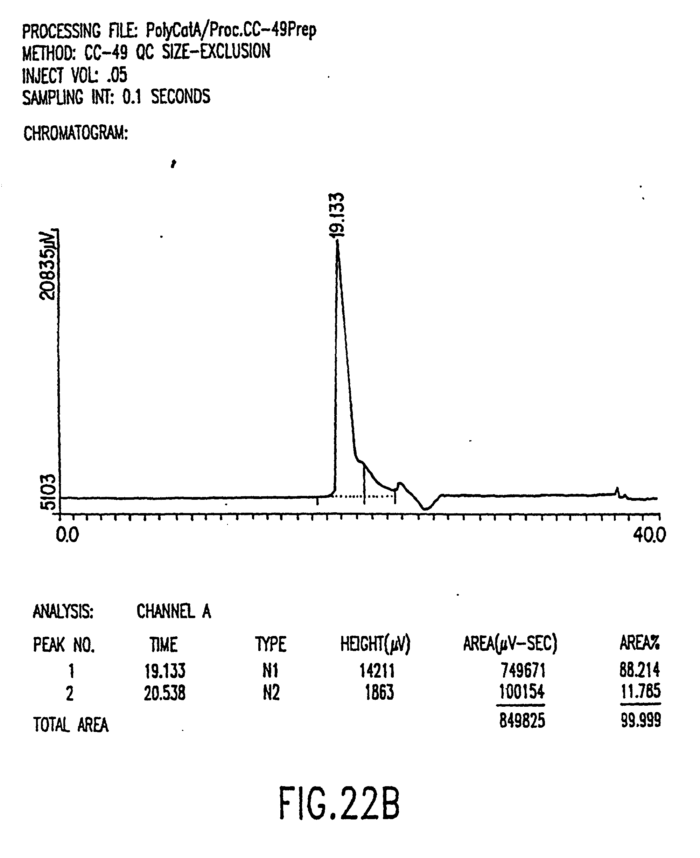

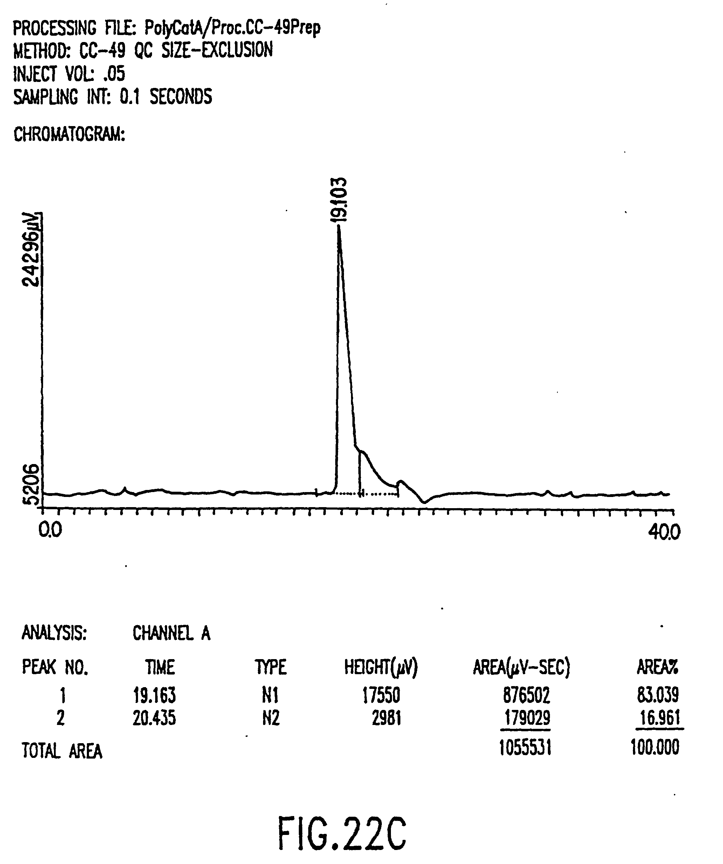

Fractions 2, 5, and 6 correspond to the three main peaks in Figure 20 and therefore were chosen to be analyzed by HPLC size exclusion. Fraction 2 corresponds to the peak that runs at 21.775 minutes in the preparative purification (Figure 20), and runs on the HPLC sizing column at 20.525 minutes, which is in the monomeric position (Figure 22A). Fractions 5 and 6 (30.1 and 33.455 minutes, respectively, in Figure 20) run on the HPLC sizing column (Figures 22B and 22C) at 19.133 and 19.163 minutes, respectively (see Table 7). Therefore, both of these peaks could be considered dimers. 40% Quenching assays were performed on all fractions of this purification. Only fraction 5 gave significant activity. 2.4 mg of active CC49 4-4-20 heterodimer Fv was recovered in fraction 5, based on the Scatchard analysis described below.

### C. N-terminal sequencing of the fractions

The active heterodimer Fv fraction should contain both polypeptide chains. N-terminal sequence analysis showed that fractions 5 and 6 displayed N-terminal sequences consistent with the prescence of both CC49 and 4-4-20 polypeptides and fraction 2 displayed a single sequence corresponding to the CC49/212/4-4-20 polypeptide only. We believe that fraction 6 was contaminated by fraction 5 (see Figure 20), since only fraction 5 had significant activity.

### D. Anti-fluorescein activity by Scatchard analysis

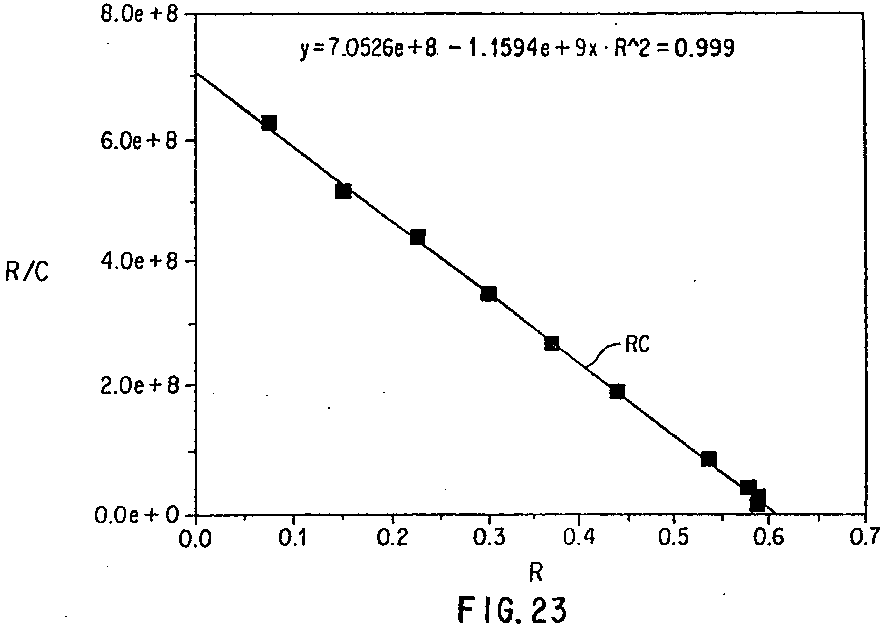

The fluorescein association constants (Ka) were determined for fractions 5 and 6 using the fluorescence quenching assay described by Herron, J.N., in *Fluorescence Hapten: An Immunological Probe,* E.W. Voss, ed., CRC Press, Boca Raton, FL (1984). Each sample was diluted to approximately 5.0 x 10⁻⁸ M with 20 mM HEPES buffer pH 8.0. 590 µl of the 5.0 x 10⁻⁸ M sample was added to a cuvette in a fluorescence spectrophotometer equilibrated at room temperature. In a second cuvette 590 µl of 20 mM HEPES buffer pH 8.0 was added. To each cuvette was added 10 µl of 3.0 x 10⁻⁷ M fluorescein in 20 mM HEPES buffer pH 8.0, and the fluorescence recorded. This is repeated until 140 µl of fluorescein had been added. The resulting Scatchard analysis for fraction 5 shows a binding constant of 1.16 x 10⁹ M⁻¹ for fraction #5 (see Figure 23). This is very close to the 4-4-20/212 sFv constant of 1.1 x 10⁹ M⁻¹ (see Pantoliano *et al*., *Biochemistry 30*:10117-10125 (1991)). The R intercept on the Scatchard analysis represents the fraction of active material. For fraction 5, 61% of the material was active. The graph of the Scatchard analysis on fraction 6 shows ' a binding constant of 3.3 x 10⁸ M⁻¹ and 14% active. The activity that is present in fraction 6 is most likely contaminants from fraction 5.

### E. Anti-TAG-72 activity by competition ELISA

The CC49 monoclonal antibody was developed by Dr. Jeffrey Schlom's group, Laboratory of Tumor Immunology and Biology, National Cancer Institute. It binds specifically to the pan-carcinoma tumor antigen TAG-72. See Muraro, R., *et al*., *Cancer Research 48*:4588-4596 (1988).

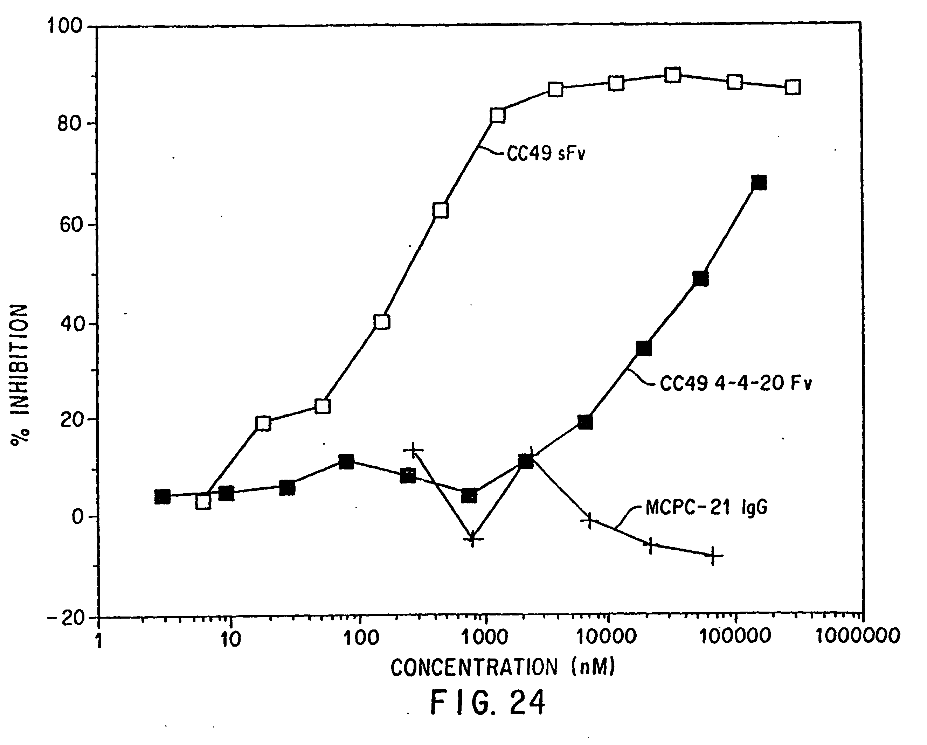

To determine the binding properties of the bivalent CC49/4-4-20 Fv (fraction 5) and the CC49/212 sFv, a competition enzyme-linked immunosorbent assay (ELISA) was set up in which a CC49 IgG labeled with biotin was competed against unlabeled CC49/4-4-20 Fv and the CC49/212 sFv for binding to TAG-72 on a human breast carcinoma extract (see Figure 24). The amount of biotin-labeled CC49 IgG was determined using a preformed complex with avidin and biotin coupled to horse radish peroxidase and O-phenylenediamine dihydrochloride (OPD). The reaction was stopped with 4N H₂SO₄ (sulfuric acid), after 10 min. and the optical density read at 490nm. This competition ELISA showed that the bivalent CC49/4-4-20 Fv binds to the TAG-72 antigen. The CC49/4-4-20 Fv needed a two hundred-fold higher protein concentration to displace the IgG than the single-chain Fv.

### Example 8

### Cross-Linking Antigen-Binding Dimers

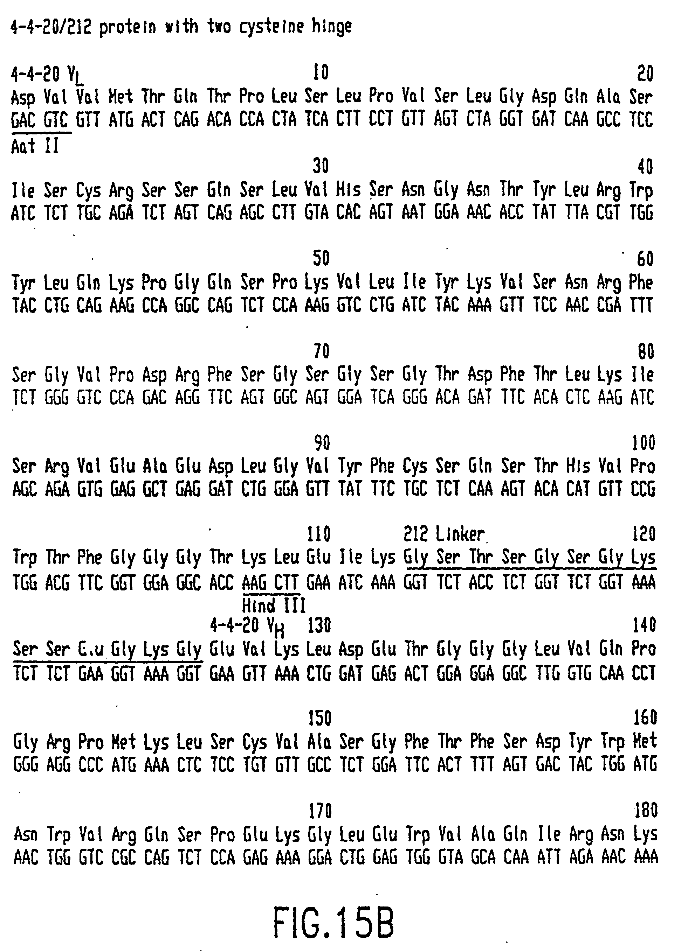

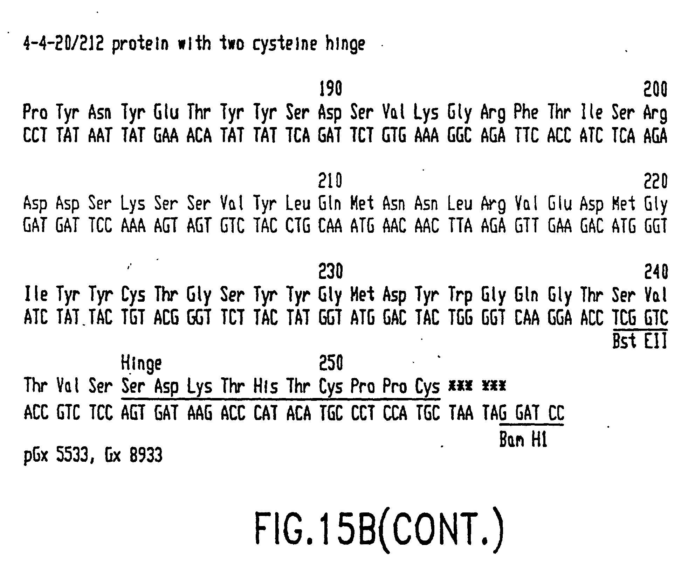

We have chemically crosslinked dimers of 4-4-20/212 antigen-binding protein with the two cysteine C-terminal extension (4-4-20/212 CPPC single-chain antigen-binding protein) in two ways. In Example 5 we describe the design and genetic construction of the 4-4-20/212 CPPC single-chain antigen-binding protein (hinge design 2 in Table 5). Figure 15B shows the nucleic acid and protein sequences of this protein. After purifying the 4-4-20/212 CPPC single-chain antigen-binding protein, using the methods described in Whitlow and Filpula, *Meth. Enzymol. 2*:97 (1991), dimers were formed by two methods. First, the free cysteines were mildly reduced with dithiothreitol (DTT) and then the disulfide-bonds between the two molecules were allowed to form by air oxidation. Second, the chemical crosslinker *bis*-maleimidehexane was used to produce dimers by crosslinking the free cysteines from two 4-4-20/212 CPPC single-chain antigen-binding proteins.

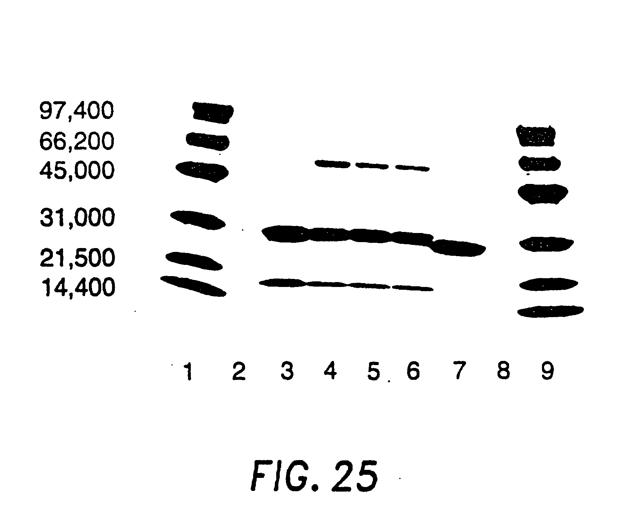

A 0.1 mg/ml solution of the 4-4-20/212 CPPC single-chain antigen-binding protein was mildly reduced using 1 mM DTT, 50 mM HEPES, 50mM NaCl, mM EDTA buffer pH 8.0 at 4°C. The samples were dialyzed against 50mM HEPES, 50 mM NaCl, 1 mM EDTA buffer pH 8.0 at 4°C overnight, to allow the oxidation of free sulfhydrals to intermolecular disulfide-bonds. Figure 25 shows a non-reducing SDS-PAGE gel after the air oxidation; it shows that approximately 10% of the 4-4-20/212 CPPC protein formed dimers with molecular weights around 55,000 Daltons.

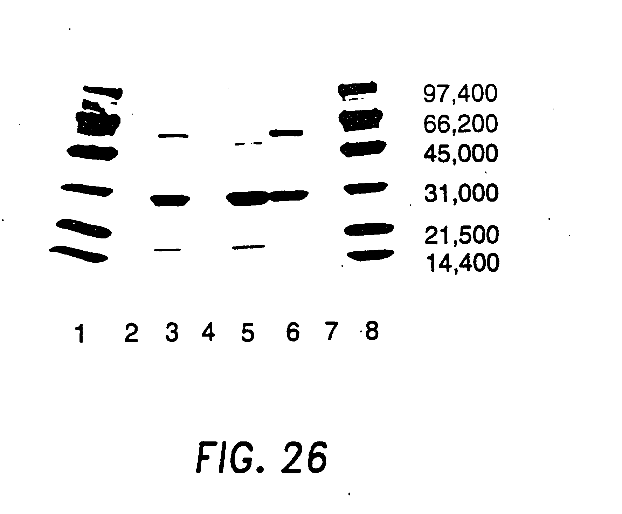

A 0.1 mg/ml solution of the 4-4-20/212 CPPC single-chain antigen-binding protein was treated with 2 mM *bis*-maleimidehexane. Unlike forming a disulfide-bond between two free cysteines in the previous example, the *bis*-maleimidehexane crosslinker material should be stable to reducing agents such as β-mercaptoethanol. Figure 26 shows that approximately 5 % of the treated material produced dimer with a molecular weight of 55,000 Daltons on a reducing SDS-PAGE gel (samples were treated with β-mercaptalethanol prior to being loaded on the gel). We further purified the *bis*-maleimidehexane treated 4-4-20/212 CPPC protein on PolyCAT A cation exchange column after the protein had been extensively dialyzed against buffer A. Figure 26 shows that we were able to enhance the fraction containing the dimer to approximately 15%.

## Claims

1. A method of producing a multivalent antigen-binding protein **characterised in that** it comprises:
(a) producing a composition comprising single-chain molecules, each single-chain molecule comprising:
(i) a first polypeptide comprising the binding portion of the variable region of an antibody heavy or light chain;
(ii) a second polypeptide comprising the binding portion of the variable region of an antibody heavy or light chain;
and
(iii) a peptide linker linking the said first and second polypeptides (i) and (ii) into the said single-chain molecule;
(b) dissociating the said single-chain molecules;
(c) re-associating the said single-chain molecules;
(d) separating multivalent antigen-binding proteins from the said single-chain molecules; and
(e) recovering the said multivalent proteins.

2. A method as claimed in claim 1 wherein the said dissociation is caused by dialysis against a dissociating solution.

3. A method as claimed in claim 1 wherein the said reassociation is caused by dialysis against a refolding solution or a refolding agent.

4. A method as claimed in claim 1 wherein the said single-chain molecules are concentrated before separating the said multivalent protein from the said single-chain molecules.

5. A method as claimed in claim 4 wherein the said concentrating occurs from approximately 0.5 mg/ml single-chain molecule to the concentration at which precipitation starts.

6. A method as claimed in any of claims 1 to 5 wherein a variable light chain of a first single-chain antigen-binding protein associates with a variable heavy chain of a second single-chain antigen-binding protein.

## Patentansprüche

1. Verfahren zur Erzeugung eines multivalenten Antigen-bindenden Proteins, **dadurch gekennzeichnet, dass** es folgendes umfasst:
(a) Erzeugen einer Zusammensetzung mit einkettigen Molekülen, wobei jedes einkettige Molekül folgendes umfasst:
(i) ein erstes Polypeptid, das den Bindungsanteil der variablen Region der schweren oder leichten Kette eines Antikörpers umfasst;
(ii) ein zweites Polypeptid, das den Bindungsanteil der variablen Region einer schweren oder leichten Kette eines Antikörpers umfasst; und
(iii) einen Peptidlinker, der die ersten und zweiten Polypeptide (i) und (ii) in dem einkettigen Molekül verbindet;
(b) Dissoziieren der einkettigen Moleküle;
(c) Reassoziieren der einkettigen Moleküle;
(d) Abtrennen multivalenter Antigen-bindender Proteine von den einkettigen Molekülen;
und
(e) Gewinnen der multivalenten Proteine.

2. Verfahren nach Anspruch 1, wobei die Dissoziierung durch eine Dialyse gegen eine dissoziierende Lösung verursacht wird.

3. Verfahren nach Anspruch 1, wobei die Reassoziierung durch eine Dialyse gegen eine Umfaltungslösung oder ein Umfaltungsmittel verursacht wird.

4. Verfahren nach Anspruch 1, wobei die einkettigen Moleküle vor dem Abtrennen des multivalenten Proteins von den einkettigen Molekülen konzentriert werden.

5. Verfahren nach Anspruch 4, wobei das Konzentrieren von ungefähr 0,5 mg/ml einkettigem Molekül bis zu der Konzentration eintritt, bei der eine Ausfällung beginnt.

6. Verfahren nach einem der Ansprüche 1 bis 5, wobei die variable leichte Kette eines ersten einkettigen Antigen-bindenden Proteins sich mit einer variablen schweren Kette eines zweiten einkettigen Antigen-bindenden Proteins assoziiert.

## Revendications

1. Procédé pour produire une protéine de liaison à l'antigène multivalente **caractérisé en ce qu'**il comprend :
(a) la production d'une composition comprenant des molécules à chaîne simple, chacune molécule à chaîne simple comprenant :
(i) un premier polypeptide comprenant la portion de liaison de la région variable d'une chaîne lourde ou légère d'un anticorps ;
(ii) un deuxième polypeptide comprenant la portion de liaison de la région variable d'une chaîne lourde ou légère d'un anticorps ;
et
(iii) un lieur peptidique liant lesdits premier et deuxième polypeptides (i) et (ii) dans ladite molécule à chaîne simple ;
(b) la dissociation desdites molécules à chaîne simple ;
(c) la réassociation desdites molécules à chaîne simple ;
(d) la séparation des protéines de liaison à l'antigène multivalentes desdites molécules à chaîne simple
et
(e) la récupération desdites protéines multivalentes.

2. Procédé selon la revendication 1, dans lequel ladite dissociation est provoquée par la dialyse avec une solution de dissociation.

3. Procédé selon la revendication 1, dans lequel ladite réassociation est provoquée par la dialyse avec une solution de repli ou un agent de repli.

4. Procédé selon la revendication 1, dans lequel lesdites molécules à chaîne simple sont concentrées avant la séparation de ladite protéine multivalente des molécules à chaîne simple.

5. Procédé selon la revendication 4, dans lequel ladite concentration se produit entre une concentration approximativement en molécule à chaîne simple de 0,5 mg/ml et la concentration à laquelle la précipitation commence.

6. Procédé selon l'une quelconque des revendications 1 à 5, dans lequel une chaîne légère variable d'une première protéine de liaison à l'antigène à chaîne simple s'associe avec une chaîne lourde variable d'une seconde protéine de liaison à l'antigène à chaîne simple.
